# EUROPEAN PATENT APPLICATION

(11) **EP 0 808 824 A2**
(43) Date of publication of application: **26.11.1997**
(21) Application number: 97303508.2
(22) Date of filing: 22.05.1997
(51) Int. Cl.: C07C 69/732, C07C 67/475, C07C 69/734, C07C 69/675, C07D 303/48, C07D 281/10

(54) **Process for preparing optically active 2-halogeno-3-hydroxypropionic acid ester**

(30) Priority: 24.05.1996 JP 128878/96
(71) Applicant: TANABE SEIYAKU CO., LTD., Chuo-ku Osaka (JP)
(72) Inventor: Kuroda, Tooru, Ashiya-shi, Hyogo-ken (JP); Yamanaka, Takeshi, Katano-shi, Osaku-fu (JP); Yamagishi, Masafumi, Amagasaki-shi, Hyogo-ken (JP); Ozaki, Yasuhiko, Neyagawa-shi, Osaka-fu (JP); Imashiro, Ritsuo, Takatsuki-shi, Osaka-fu (JP)
(74) Representative: Hardisty, David Robert

(57) **Abstract**

A novel process for preparing optically active 2-halogeno-3-hydroxypropionic acid ester compound (III), which comprises reacting the aldehyde compound (I) with the silylketene acetal compound (II) in the presence of a chiral Lewis acid, as illustrated below. 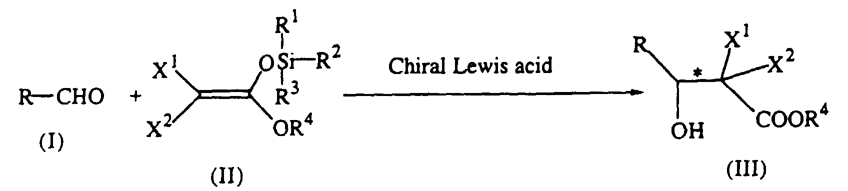 wherein R is aryl, cycloalkyl, etc., one of X¹ and X² is H, and the other is Cl or Br, or both are Cl or Br, R¹, R² and R³ are the same or different, and are lower alkyl or aryl, R⁴ is lower alkyl or aryl, and * is asymmetric carbon atom. The compound (III) is very useful as an intermediate for preparing medicament such as diltiazem hydrochloride.

## Description

The present invention relates a novel process for preparing an optically active 2-halogeno-3-hydroxypropionic acid ester compound, which is useful as an intermediate for preparing medicaments.

Optically active 2-halogeno-3-hydroxypropionic acid ester compounds are important as an intermediate for preparing various medicaments such as diltiazem hydrochloride (chemical name: (2S,3S)-3-acetoxy-5-[2-(dimethylamino)ethyl]-2,3-dihydro-2-(4-methoxyphenyl)-1,5-benzothiazepin-4(5H)-one hydrochloride), which is useful as a calcium channel blocker.

Hitherto, the following processes for preparing optically active 2-halogeno-3-hydroxypropionic acid ester compounds have been disclosed:
(1) a process comprising reacting a halogenoacetic acid ester with a benzaldehyde compound in the presence of a chiral lithium amide compound and an alkyl lithium (cf., JP-A-1-226881);
(2) a process comprising subjecting a 2-halogeno-3-oxopropionic acid ester compound to asymmetric reduction with an alkali metal borohydride or an alkali metal aluminum hydride in the presence of a chiral amino acid derivative and a lower aliphatic alcohol (cf., JP-A-3-190865);
(3) a process comprising subjecting a 2-halogeno-3-oxopropionic acid ester compound to asymmetric reduction with a microorganism having an ability of asymmetrically reducing a carbonyl group (cf., JP-A-3-272691); and
(4) a process comprising reacting a halogenoacetic acid t-butyl ester with a benzaldehyde compound in the presence of a chiral boron compound (cf., Tetrahedron Letters, **32**, 2857-2860 (1991)).

However, the above processes (1) to (4) have some defects, for example, these processes need expensive reagents, and comprise many complicated steps, so that these processes are not suitable for the production on industrial scale. Under the circumstances, it is desired to develop an industrially advantageous novel process for preparing optically active 2-halogeno-3-hydroxypropionic acid ester compounds.

Recently, there have been reported processes for preparing a 3-hydroxypropionic acid ester compound of high optical purity, which comprise reacting an aldehyde compound with a silylketene acetal compound which is derived from an acetic acid ester compound, in the presence of a chiral Lewis acid, for example,
a process comprising using a chiral titanium compound as a chiral Lewis acid (E. M. Carreira et al., J. Am. Chem. Soc., 1994, **116**, 8837; G. E. Keck et al., J. Am. Chem. Soc., 1995, **117**, 2363),
a process comprising using a chiral boron compound (S. Kiyooka et al., J. Org. Chem., 1991, **56**, 2276; S. Masamune et al., J. Am. Chem. Soc., 1991, **113**, 9365; H. Yamamoto et al., Synlett, 1991, 439; M.T. Reetz, Tetrahedron Lett., 1986, **27**, 4721),
a process comprising using a chiral copper compound (D.A. Evans, J. Am. Chem. Soc., 1996, **118**, 5814),
a process comprising using a chiral aluminum compound (M.T. Reetz, Chem. Ind., 824, 1986), and
a process comprising using a chiral tin compound (T. Mukaiyama, J. Am. Chem. Soc., 1991, **113**, 4247).

However, when reacting an aldehyde compound with a silylketene acetal compound which is derived from a halogenoacetic acid ester compound (e.g., chloroacetic acid ester, dichloroacetic acid ester, etc.) in the presence of the above-mentioned chiral Lewis acid, it cannot be expected that a 2-halogeno-3-hydroxypropionic acid ester compound of high optical purity is prepared because said silylketene acetal compound has a highly electronegative and sterically bulky halogen atom in the molecular thereof.

Besides, there are some reports concerning the conversion of a chloroacetic acid ester or a dichloroacetic acid ester into a silylketene acetal (T. Nakai et al., Tetrahedron Asymmetry, 1991, **2**, 993; G. Simchen, Liebigs Ann. Chem., 1983, 816; C.S. Wilcox et al., Tetrahedron Lett., 1984, **25**, 699; C. Lion et al., Bull. Chim. Belg., 1988, **97**, 227; V.V. Schepin et al., Zh. Obshch. Khim., 1990, **60**, 2805). However, these processes have many defects, for example, silylating agents and bases used therein are expensive; a yield of the desired product is low; and undesirable C-silyl compound is unnecessarily prepared, in addition to the desired O-silyl compound.

In addition, Tetrahedron Letters, **38**, 1447-1448 (1997) discloses also a process for preparing an optically active 2-halogeno-3-hydroxypropionic acid ester compound by reacting an aldehyde compound with a difluorosilylketene acetal compound in the presence of a chiral boron compound. However, when using a chloro- or bromo-containing silylketene acetal compound instead of a fluorine-containing silylketene acetal compound, the stereocircumstances or the reactivity of the compounds are quite different based on the differences in atomic radius and in electronegativity between fluorine atom and chlorine atom (or bromine atom). Therefore, it is not expected that the optical activity of the reaction products will be controlled in similar way and then the asymmetric reaction will proceed similarly between the process of the above literature and the present invention.

An object of the present invention is to provide a process for preparing an optically active 2-halogeno-3-hydroxypropionic acid ester compound of high optical purity by reacting an aldehyde compound with a silylketene acetal compound in the presence of a chiral Lewis acid.

According to the present invention, an optically active 2-halogeno-3-hydroxypropionic acid ester compound of the formula (III): 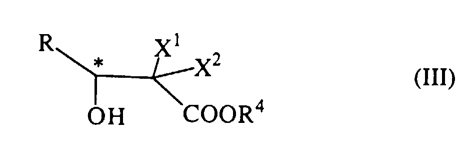 wherein R is a lower alkyl group, a lower alkenyl group, an aryl group or a cycloalkyl group, R⁴ is a lower alkyl group or an aryl group, one of X¹ and X² is a hydrogen atom, and the other is a chlorine atom or a bromine atom, or both are a chlorine atom or a bromine atom, and * means an asymmetric carbon atom, may be prepared by reacting an aldehyde of the formula (I):

R-CHO (I)

wherein R is the same as defined above, with a silylketene acetal compound of the formula (II): 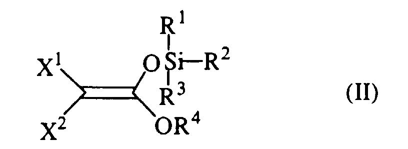 wherein R¹, R² and R³ are the same or different and each are a hydrogen atom, a lower alkyl group or an aryl group, and R⁴, X¹ and X² are the same as defined above, in the presence of a chiral Lewis acid.

Suitable examples of the aldehyde compounds (I) are compounds of the formula (I) wherein R is a lower alkyl group (e.g., methyl, ethyl, n-propyl, isopropyl, t-butyl, n-butyl), a lower alkenyl group (e.g., vinyl, propenyl, butenyl), an aryl group (e.g., phenyl, naphthyl), or a cycloalkyl group (e.g., cyclopropyl, cyclobutyl, cyclopentyl). The aryl group such as phenyl and naphthyl may optionally have a substituent selected from a halogen atom (e.g., fluorine, chlorine, bromine), a lower alkyl group (e.g., methyl, ethyl, n-propyl, isopropyl, t-butyl, n-butyl), and a lower alkoxy group (e.g., methoxy, ethoxy, n-propyloxy, isopropyloxy, t-butyloxy, n-butyloxy).

Among these compounds, the more preferred aldehyde compounds (I) are compounds of the formula (I) wherein R is a lower alkylphenyl group or a lower alkoxyphenyl group, and the most preferred compound (I) are compounds of the formula (I) wherein R is a 4-methylphenyl group or a 4-methoxyphenyl group.

Suitable examples of the silylketene acetal compounds (II) are compounds of the formula (II) wherein one of X¹ and X² is a hydrogen atom, and the other is a chlorine atom or a bromine atom, or both are a chlorine atom or a bromine atom, R¹, R² and R³ are the same or different and each are a hydrogen atom, a lower alkyl group (e.g., methyl, ethyl, n-propyl, isopropyl, t-butyl, n-butyl) or an aryl group (e.g., phenyl, naphthyl), and R⁴ is a lower alkyl group (e.g., methyl, ethyl, n-propyl, isopropyl, t-butyl, n-butyl) or an aryl group (e.g., phenyl, naphthyl). The aryl group for R¹, R² and R³ may optionally be substituted by a group selected from a lower alkyl group (e.g., methyl, ethyl, n-propyl, isopropyl, t-butyl, n-butyl), and the aryl group for R⁴ may optionally have a substituent selected from a halogen atom (e.g., fluorine, bromine, chlorine), a lower alkyl group (e.g., methyl, ethyl, n-propyl, isopropyl, t-butyl, n-butyl), a lower alkoxy group (e.g., methoxy, ethoxy, n-propyloxy, isopropyloxy, t-butyloxy, n-butyloxy), a halogeno-lower alkyl group (e.g., trifluoromethyl), a di-lower alkylamino group (e.g., dimethylamino, diethylamino), a nitro group and a cyano group.

Among these silylketene acetal compounds (II), the preferred compounds (II) are compounds of the formula (II) wherein one of X¹ and X² is a hydrogen atom, and the other is a bromine atom or a chlorine atom, or both are a chlorine atom or a bromine atom, R¹, R² and R³ are the same, and are a methyl group, an ethyl group, n-propyl group, an isopropyl group, or an n-butyl group, or among these R¹, R² and R³, two groups are a methyl group, and the other is a t-butyl group; two groups are a methyl group, and the other is an isopropyl group; two groups are a methyl group, and the other is a phenyl group; two groups are a methyl group, and the other is a 1,1,2,2-tetramethylpropyl group; or two groups are a methyl group, and the other is a hydrogen atom, and R⁴ is a methyl group, an ethyl group or a n-propyl group. Among these compounds, the more preferred compounds (II) are compounds of the formula (II) wherein one of X¹ and X² is a hydrogen atom, and the other is a chlorine atom, or both are a chlorine atom, R¹, R² and R³ are the same methyl group, ethyl group, n-propyl group or n-butyl group, and R⁴ is a methyl group, an ethyl group or an n-propyl group. Especially preferable compounds (II) are compounds of the formula (II) wherein one of X¹ and X² is a hydrogen atom, and the other is a chlorine atom, or both are a chlorine atom, all of R¹, R², R³ and R⁴ are a methyl group, or R¹, R² and R³ are a methyl group, and R⁴ is an ethyl group.

The chiral Lewis acid used in the present invention includes a chiral Lewis acid which can be used in aldol reaction, for example, a chiral complex consisting of a ligand, and a boron or a metal selected from titanium, copper, aluminum and tin.

The ligand forming the above-mentioned chiral complex includes the following compounds:
(1) 1,2,3,4-tetrahydroxybutane having substituents on 1- and 4-positions, wherein 2- and 3-hydroxy groups may be protected, and the substituents for 1- and 4-positions are, for example, a lower alkyl group, a lower alkylphenyl group, a lower alkylnaphthyl group, or a cyclolalkyl group, and the protecting group for 2- and 3-hydroxy groups are, for example, a lower alkylene group formed by combining the 2- and 3-positions;
(2) [1,1'-binaphthalene]-2,2'-diol having optionally substituents and the substituents are, for example, 1 to 2 substituents selected from a halogen atom and a lower alkyl group;
(3) α-amino acid having a substituent on the carbon atom at α-position, wherein the α-amino group may be protected, and the substituent is, for example, a lower alkyl group having optionally 1 or 2 substituents selected from a carboxyl group and a phenylcarbonyloxy group having optionally 1 or 2 substituents selected from a lower alkyl group and a lower alkoxy group, a phenyl-lower alkyl group having optionally a lower alkyl substituent, and the protecting group is, for example, a phenylsulfonyl group or naphthylsulfonyl group which may be substituted by a nitro group, a halogen atom, a lower alkyl group or a lower alkoxy group;
(4) α-hydroxycarboxylic acid having a substituent on the carbon atom at α-position, wherein the substituent is, for example, a lower alkyl group having optionally 1 to 2 substituents selected from a carboxyl group and a phenylcarbonyloxy group having optionally 1 or 2 substituents selected from a lower alkyl group and a lower alkoxy group, or a phenyl-lower alkyl group having optionally a lower alkyl substituent;
(5) 1-protected or unprotected amino-2-hydroxycycloalkane having optionally a substituent on 1- and 2-positions, wherein the substituent is, for example, a lower alkyl group or a phenyl-lower alkyl group, and the protecting group is, for example, a lower alkylsulfonyl group, a trifluoromethanesulfonyl group, a lower alkylphenylsulfonyl group, or a lower alkylnaphthysulfonyl group;
(6) bridged 1,2-dihydroxycycloalkane having optionally a substituent wherein the substituent is, for example, a lower alkyl group, a lower alkylphenyl group, a lower alkylnaphthyl group, and the bridged cycloalkane group is, for example, a cycloalkane ring which is bridged by a lower alkylene group; and
(7) 1,2-dihydroxycycloalkane having a substituent on 1- and/or 2-positions, wherein the substituent is, for example, a lower alkyl group, a lower alkylphenyl group, or a lower alkylnaphthyl group.

The chiral Lewis acid includes, for example, the following compounds:
(1) a chiral titanium compound of the formula (IV): 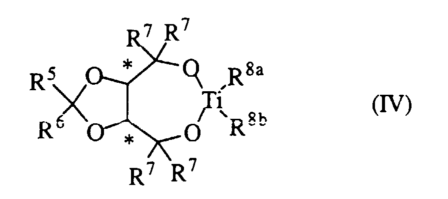 wherein R⁵ and R⁶ are the same or different and each are a hydrogen atom, a lower alkyl group or an aryl group, or both combine each other to form a lower alkylene group or an oxo group, R⁷ is a lower alkyl group, an aryl group or a cycloalkyl group, R^{8a} and R^{8b} are the same or different and each are a halogen atom, a trifluoromethanesulfonyloxy group or a lower alkoxy group, and * means an asymmetric carbon atom, and the aryl group for R⁵, R⁶ and R⁷ may be a phenyl group or naphthyl group which may optionally be substituted by a halogen atom (e.g., fluorine, chlorine, bromine), a lower alkyl group (e.g., methyl, ethyl, n-propyl, isopropyl, t-butyl, n-butyl), a halogeno-lower alkyl group (e.g., trifluoromethyl group), a lower alkoxy group (e.g., methoxy, ethoxy, n-propyloxy, isopropyloxy, t-butyloxy, n-butyloxy), or a phenyl group;
(2) a chiral titanium compound of the formula (V-a) or (V-b): 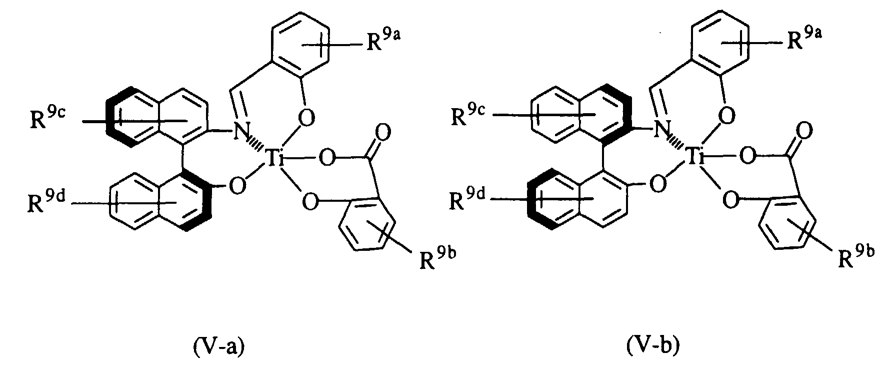 wherein R^{9a}, R^{9b}, R^{9c} and R^{9d} are the same or different and each are a hydrogen atom, 1 or 2 halogen atoms or a lower alkyl group;
(3) a chiral titanium compound of the formula (VI-a) or (VI-b): 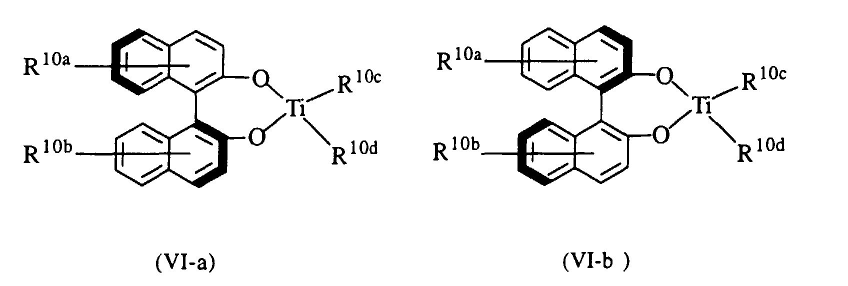 wherein R^{10a} and R^{10b} are the same or different and each are a hydrogen atom, 1 or 2 halogen atoms or a lower alkyl group, and R^{10c} and R^{10d} are the same or different and each are a halogen atom, a trifluoromethanesulfonyloxy group or a lower alkoxy group;
(4) a chiral boron compound of the formula (VII): 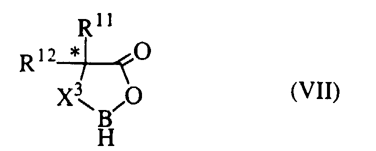 wherein X³ is an oxygen atom or a lower alkylsulfonyl-substituted nitrogen atom, a trifluoromethanesulfonyl-substituted nitrogen atom, an arylsulfonyl-substituted nitrogen atom, R¹¹ and R¹² are different, and independently a hydrogen atom, a substituted or unsubstituted lower alkyl group or an aralkyl group, or both combine each other to form a substituted or unsubstituted lower alkylene group, and * means an asymmetric carbon atom, and the aryl moiety of the arylsulfonyl-substituted nitrogen atom for X³ is a phenyl group or naphthyl group which may be substituted by a nitro group, a cyano group, a lower alkoxycarbonyl group (e.g., methoxycarbonyl, ethoxycarbonyl, n-propyloxycarbonyl, isopropyloxycarbonyl, t-butyloxycarbonyl, n-butyloxycarbonyl), in addition to the same substituents on the phenyl group or naphthyl group for R⁵, R⁶ and R⁷, and the aralkyl group for R¹¹ and R¹² is, for example, a phenyl-lower alkyl group or naphthyl-lower alkyl group which may optionally be substituted by a lower alkyl group (e.g., methyl, ethyl, n-propyl, isopropyl, t-butyl, n-butyl) or a lower alkoxy group (e.g., methoxy, ethoxy, n-propyloxy, isopropyloxy, t-butyloxy, n-butyloxy). The substituent on the lower alkyl group for R¹¹ and R¹² is, for example, 1 or 2 substituents selected from a carboxyl group, and a phenylcarbonyloxy group which may optionally be substituted by 1 or 2 substituents selected from a lower alkyl group and a lower alkoxy group, and the substituent on the lower alkylene group is, for example, 1 or 2 lower alkyl groups;
(5) a chiral copper compound of the formula (VIII): 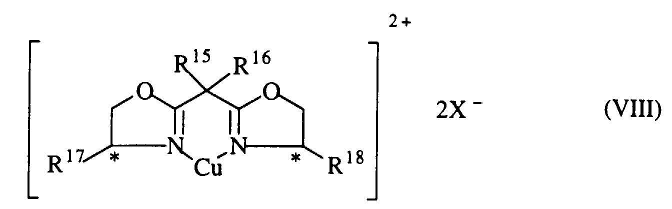 wherein R¹⁵, R¹⁶, R¹⁷ and R¹⁸ are the same or different and each are a lower alkyl group, X⁻ is a counter anion, and * means an asymmetric carbon atom;
(6) a chiral aluminum compound of the formula (IX): 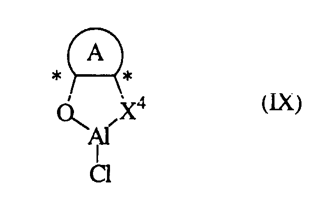 wherein X⁴ is an oxygen atom or an arylsulfonyl-substituted nitrogen atom, Ring A is a substituted or unsubstituted bridged cycloalkane ring, a substituted or unsubstituted cycloalkane ring, and * means an asymmetric carbon atom, provided that when X⁴ is an oxygen atom, then Ring A is not an unsubstituted cycloalkane ring, and the aryl moiety of the arylsulfonyl-substituted nitrogen atom for X⁴ is a phenyl group or naphthyl group which may optionally be substituted by the same substituents for the aryl moiety of the arylsulfonyl-substituted nitrogen atom for X³, and the substituent on the bridged cycloalkane ring or cycloalkane ring for Ring A is, for example, a lower alkyl group (e.g., methyl, ethyl, n-propyl, isopropyl, t-butyl, n-butyl), a lower alkylphenyl group, a lower alkylnaphthyl group, and the bridged cycloalkane ring is, for example, a cycloalkane ring which is bridged by a lower alkylene group; and
(7) a chiral tin compound of the formula (X): 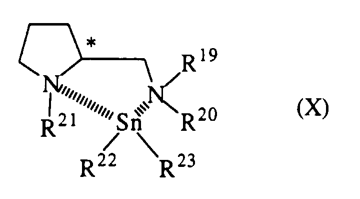 wherein R¹⁹ and R²⁰ are different, and independently a hydrogen atom or an aryl group, or both combine each other to form a substituted or unsubstituted lower alkylene group, R²¹ is a lower alkyl group, R²² and R²³ are a trifluoromethanesulfonyloxy group or a chlorine atom, and * means an asymmetric carbon atom, and the aryl group for R¹⁹ and R²⁰ is, for example, a phenyl group or naphthyl group which may optionally be substituted by a lower alkyl group (e.g., methyl, ethyl, propyl, isopropyl, t-butyl, n-butyl), and the substituent on the lower alkylene group for R¹⁹ and R²⁰ is, for example, a lower alkyl group, or a lower alkoxy-lower alkyl group.

When the silylketene acetal compound (II) is a monohalogeno compound (one of X¹ and X² is a hydrogen atom, and the other is a chlorine atom or a bromine atom), then the chiral Lewis acid is preferably a chiral titanium compound. When the silylketene acetal compound (II) is a dihalogeno compound (both of X¹ and X² are a chlorine atom or a bromine atom), then the chiral Lewis acid is preferably a chiral boron compound.

The chiral titanium compound (IV) includes, for example, compounds of the formula (IV) wherein R⁵ and R⁶ are the same or different and each are a hydrogen atom, a lower alkyl group (e.g., methyl, ethyl, n-propyl, isopropyl, t-butyl, n-butyl), an aryl group (e.g., phenyl, naphthyl), or both combine each other to form a lower alkylene group (e.g., methylene, ethylene, trimethylene, propylene, tetramethylene), or an oxo group, R⁷ is a lower alkyl group (e.g., methyl, ethyl, n-propyl, isopropyl, t-butyl, n-butyl), an aryl group (e.g., phenyl, naphthyl), a cycloalkyl group (e.g., cyclopropyl, cyclobutyl, cyclopentyl), R^{8a} and R^{8b} are the same or different and each are a halogen atom (e.g., fluorine, bromine, chlorine), a trifluoromethanesulfonyloxy group, or a lower alkoxy group (e.g., methoxy, ethoxy, n-propyloxy, isopropyloxy, t-butyloxy, n-butyloxy). The aryl group for R⁵ and R⁶ may optionally be substituted by a lower alkyl group (e.g., methyl, ethyl, n-propyl, isopropyl, t-butyl, n-butyl). The aryl group for R⁷ may be a phenyl group or naphthyl group which may optionally be substituted by 1 or 2 groups selected from a halogen atom, a trifluoromethyl group, a phenyl group and a lower alkyl group, and among them, a lower alkyl group-substituted phenyl group is preferable.

Among the chiral titanium compounds (IV), more preferable compounds are compounds of the formula (IV) wherein R⁵ and R⁶ are the same lower alkyl group, R⁷ is a phenyl group or naphthyl group which may optionally be substituted by 1 or 2 groups selected from a halogen atom, a trifluoromethyl group, a phenyl group and a lower alkyl group, and R^{8a} and R^{8b} are a halogen atom. Especially preferable compounds are compounds of the formula (IV) wherein both of R⁵ and R⁶ are a methyl group, R7 is a methylphenyl group, and both of R^{8a} and R^{8b} are a chlorine atom.

The chiral titanium compound (IV) is chiral titanium compounds of the following formula (IV-a) or (IV-b), and may be selected according to the configuration of the 3-position of the desired 2-halogeno-3-hydroxypropionic acid ester (III). 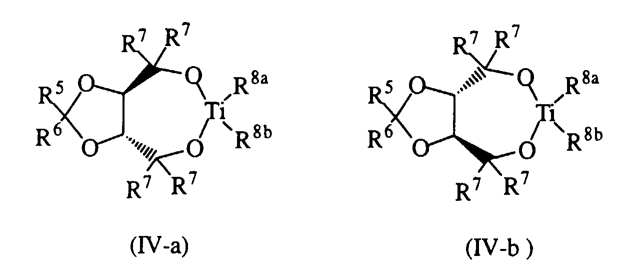 wherein R⁵, R⁶, R⁷, R^{8a} and R^{8b} are the same as defined above.

The chiral boron compound (VII) is compounds of the formula (VII) wherein X³ is oxygen atom or a lower alkylsulfonyl-substituted nitrogen atom, a trifluoromethanesulfonyl-substituted nitrogen atom, or an arylsulfonyl-substituted nitrogen atom such as a phenylsulfonyl-substituted nitrogen atom, a naphthylsulfonyl-substituted nitrogen atom, R¹¹ and R¹² are different, and independently a hydrogen atom; a substituted or unsubstituted lower alkyl group (e.g., methyl, ethyl, n-propyl, isopropyl, t-butyl, n-butyl); an aralkyl group such as a phenyl-lower alkyl group, a naphthyl-lower alkyl group, or both combine each other to form a lower alkylene group (e.g., methylene, ethylene, trimethylene, tetramethylene) having optionally 1 or 2 lower alkyl substituents.

The substituent on the lower alkyl group for R¹¹ and R¹² is, for example, 1 or 2 groups selected from a carboxyl group and a phenylcarbonyloxy group which may optionally be substituted by 1 or 2 groups selected from a lower alkyl group and a lower alkoxy group. The aryl moiety of the arylsulfonyl-substituted nitrogen atom for X³ is, for example, a phenyl group or naphthyl group which may optionally be substituted by a nitro group; a cyano group; a halogen atom (e.g., fluorine, bromine, chlorine); a lower alkyl group (e.g., methyl, ethyl, n-propyl, isopropyl, t-butyl, n-butyl); a lower alkoxy group (e.g., methoxy, ethoxy, n-propyloxy, isopropyloxy, t-butyloxy, n-butyloxy); a halogeno-lower alkyl group (e.g., trifluoromethyl); a lower alkoxycarbonyl group (e.g., methoxycarbonyl, ethoxycarbonyl, n-propyloxycarbonyl, isopropyloxycarbonyl, t-butyloxycarbonyl, n-butyloxycarbonyl); or phenyl group. The aralkyl group for R¹¹ and R¹² is, for example, a phenyl-lower alkyl group or naphthyl-lower alkyl group which may optionally be substituted by a lower alkyl group (e.g., methyl, ethyl, n-propyl, isopropyl, t-butyl, n-butyl), or a lower alkoxy group (e.g., methoxy, ethoxy, n-propyloxy, isopropyloxy, t-butyloxy, n-butyloxy).

Among the chiral boron compounds (VII), the preferable compound (VII) are compounds of the formula (VII) wherein X³ is a nitrogen atom substituted by a phenylsulfonyl group or naphthylsulfonyl group which may optionally be substituted by 1 to 3 groups selected from a nitro group, a halogen atom, a lower alkyl group, a halogeno-lower alkyl group and a lower alkoxy group, R¹¹ and R¹² are different, and independently a hydrogen atom, a lower alkyl group or a phenyl-lower alkyl group, and the more preferable compounds (VII) are compounds of the formula (VII) wherein X³ is a nitrogen atom substituted by a phenylsulfonyl group or naphthylsulfonyl group which may optionally be substituted by 1 to 3 groups selected from a nitro group, a chlorine atom, a methyl group, a trifluoromethyl group and a methoxy group, and R¹¹ and R¹² are different, and independently a hydrogen atom, an isopropyl group, an isobutyl group, a sec-butyl group, a tert-butyl group or a benzyl group.

The most preferable compounds (VII) are compounds of the formula (VII) wherein X³ is a nitrogen atom substituted by a nitrophenylsulfonyl group, and R¹¹ and R¹² are different and independently a hydrogen atom or an isopropyl group.

The amount of the chiral Lewis acid is not critical, but it is usually in the range of 1 to 200 mole %, preferably in the range of 10 to 120 mole %, to the amount of the compound (I).

The solvent used in the present invention may be any one which does not disturb the reaction, for example, ethers (e.g., dioxane, tetrahydrofuran), nitriles (e.g., acetonitrile), aromatic hydrocarbons (e.g., benzene, toluene, xylene, mesitylene), halogenated aromatic hydrocarbons (e.g., chlorobenzene, o-dichlorobenzene, m-dichlorobenzene, p-dichlorobenzene, trichlorobenzene), halogenated aliphatic hydrocarbons (e.g., methylene chloride, chloroform, carbon tetrachloride, 1,2-dichloroethane), aliphatic hydrocarbons (e.g., n-hexane, n-heptane), alicyclic hydrocarbons (e.g., cyclohexane), and aprotic polar solvents (e.g., N,N-dimethylformamide, dimethylsulfoxide, nitroethane). Among these solvents, aromatic hydrocarbons and halogenated aliphatic hydrocarbons are preferable, and toluene and methylene chloride are especially preferable.

These solvents may be used alone but can be used in the form of a mixture of two or more solvents thereof.

The reaction is carried out at a temperature between -100°C and 20°C, preferably at a temperature between -78°C and -10°C.

Among the optically active 2-halogeno-3-hydroxypropionic acid ester compound (III) thus obtained, the monohalogeno compound (one of X¹ and X² is a hydrogen atom, and the other is a chlorine atom or a bromine atom) can be converted into a corresponding optically active trans-2,3-epoxypropionic acid ester compound of the formula (XI): 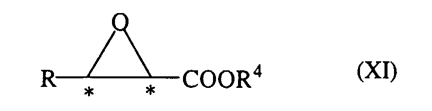 wherein R is a lower alkyl group, a lower alkenyi group, an aryi group or a cycloalkyl group, R⁴ is a lower alkyl group or an aryl group, and * means an asymmetric carbon atom, by the method disclosed in JP-A-3-190865 or JP-A-3-56471, i.e., by subjecting the compound (III) to intramolecular cyclization reaction. The cyclization reaction is carried out in the presence of a base (e.g., an alkali metal alkoxide, 1,8-diazabicyclo[5.4.0]-7-undecene, etc.) in a suitable solvent (e.g., a lower alkanol) at a temperature between -10°C and 50°C, preferably at a temperature between 0°C and room temperature.

In this reaction, the optically active 3S-type compound (III), i.e., optically active (2R,3S)- and (2S,3S)-2-halogeno-3-hydroxypropionic acid ester compound (III) can be converted into (2R,3S)-trans-2,3-epoxypropionic acid ester compound (XI) by intramolecular cyclization reaction, regardless of the configuration at the 2-position. On the other hand, the optically active 3R-type compound (III), i.e., optically active (2R,3R)- and (2S,3R)-2-halogeno-3-hydroxypropionic acid ester compound (III) can be converted into (2S,3R)-trans-2,3-epoxypropionic acid ester compound (XI) by intramolecular cyclization reaction, regardless of the configuration at the 2-position.

When converting the dihalogeno compound (III) (i.e., both of X¹ and X² are a chlorine atom or a bromine atom) into the compound (XI), the compound (III) should be reduced by a conventional method into a monochloro or a monobromo compound (III) prior to the intramolecular cyclization reaction. This conversion reaction is readily carried out by stirring the dihalogeno compound (III) (i.e., both of X¹ and X² are a chlorine atom or a bromine atom) in a mixture of zinc and acetic acid at room temperature for about 1.5 hour (cf., Chem. Pharm. Bull., **41** (4), 643-648, 1993).

When using the optically active trans-2,3-epoxypropionic acid ester compound (XI) thus obtained, an optically active 1,5-benzothiazepine derivative, which is useful as a medicament, can be prepared, for example, by a conventional process as shown in the following reaction scheme. 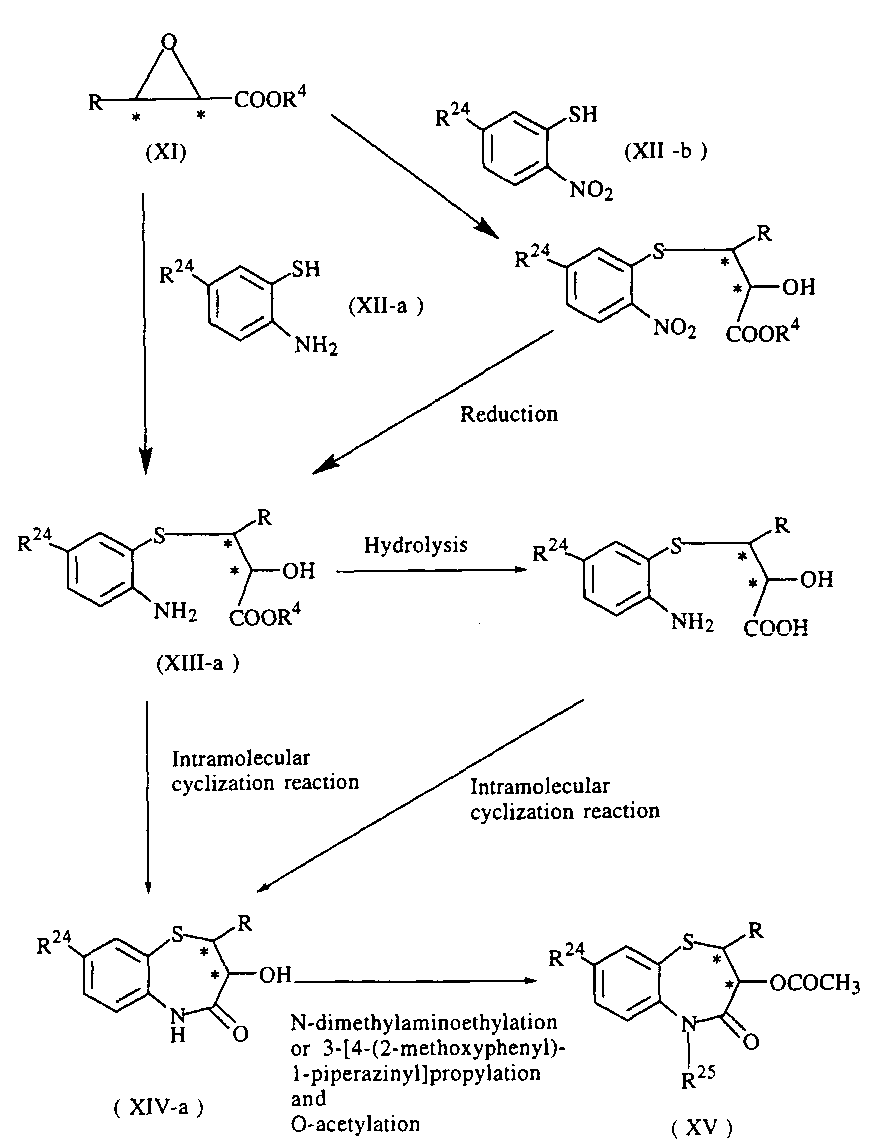 wherein R²⁴ is a hydrogen atom, a halogen atom or a lower alkyl group, R²⁵ is a 2-dimethylaminoethyl group or a 3-[4-(2-methoxyphenyl)-1-piperazinyl]propyl group, and R, R⁴ and * are the same as defined above.

That is, the optically active cis-3-acetoxy-1,5-benzothiazepine compound (XV) is prepared by
(i) reacting an optically active trans-2,3-epoxypropionic acid ester compound (XI) with an aminothiophenol compound (XII-a), or with a nitrothiophenol compound (XII-b) and reducing the nitro group of the product, to give an optically active threo-3-(2-aminophenylthio)-2-hydroxypropionic acid ester compound (XIII-a),
(ii) if necessary, hydrolyzing the resulting compound (XIII-a),
(iii) subjecting the product obtained by the hydrolysis of the compound (XIII-a), or the compound (XIII-a) per se, to intramolecular cyclization reaction to give an optically active cis-3-hydroxy-1,5-benzothiazepine compound (XIV-a),
(iv) then, followed by introducing a 2-dimethylaminoethyl group or a 3-[4-(2-methoxyphenyl)-1-piperazinyl]propyl group onto the 5-nitrogen atom, and acetylating the 3-hydroxy group of the compound (XIV-a), or vice versa.

The above process can be carried out by the method disclosed in JP-A-60-202871, JP-A-2-286672, JP-A-3-157378, JP-A-4-234866, JP-A-5-201865, JP-B-46-16988, JP-B-46-43785, JP-B-47-813, JP-B-53-18038, JP-B-63-13994, JP-B-63-17832 or JP-B-46-8982.

When using (2R,3S)-trans-2,3-epoxypropionic acid ester compound (XI) which is prepared from (3S)-2-halogeno-3-hydroxypropionic acid ester compound (III), (2S,3S)-cis-3-acetoxy-1,5-benzothiazepine compound (XV) can be prepared via (2S,3S)-threo-3-(2-aminophenylthio)-2-hydroxypropionic acid ester compound (XlII-a).

On the other hand, when using (2S,3R)-trans-2,3-epoxypropionic acid ester compound (XI) which is prepared from (3R)-2-halogeno-3-hydroxypropionic acid ester compound (III), (2R,3R)-cis-3-acetoxy-1,5-benzothiazepine compound (XV) can be prepared via (2R,3R)-threo-3-(2-aminophenylthio)-2-hydroxypropionic acid ester compound (XIII-a).

Besides, using the optically active trans-2,3-epoxypropionic acid ester compound (XI), an optically active 1,5-benzothiazepine derivative, which is useful as a medicament, can be prepared by a conventional process as shown in the following reaction scheme. 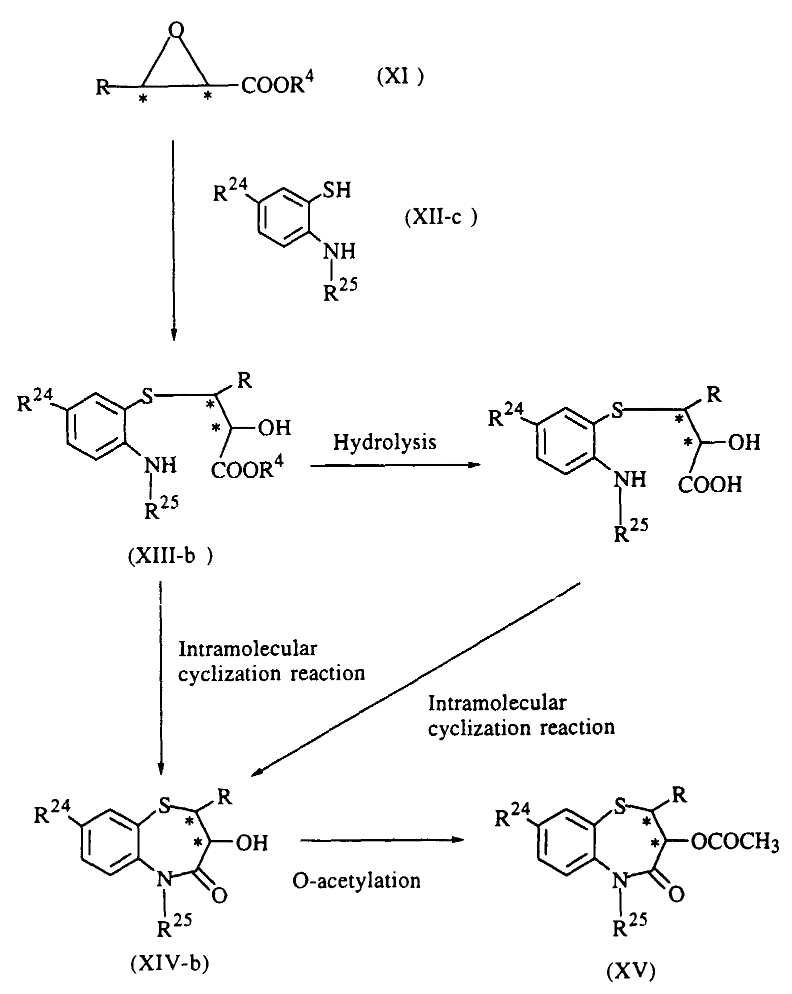 wherein R, R⁴, R²⁴, R²⁵ and * are the same as defined above.

That is, the optically active cis-3-acetoxy-1,5-benzothiazepine compound (XV) is prepared by reacting the optically active trans-2,3-epoxypropionic acid ester compound (XI) with the N-alkylaminothiophenol compound (XII-c), if necessary, hydrolyzing the resulting optically active threo-3-[2-(N-alkylamino)phenylthio]-2-hydroxypropionic acid ester compound (XIII-b), subjecting the hydrolyzed product or the compound (XIII-b) to intramolecular cyclization reaction, followed by acetylating the 3-hydroxy group of the resulting optically active cis-5-alkyl-3-hydroxy-1,5-benzothiazepine compound (XIV-b). The above process is carried out by the method disclosed in JP-A-6-279398.

The cis-3-acetoxy-1,5-benzothiazepine compounds (XV) are preferably compounds of the formula (XV) wherein
(a) R is a 4-methoxyphenyl group, R²⁴ is a hydrogen atom, R²⁵ is a 2-dimethylaminoethyl group, and the configuration is (2S,3S)-configuration;
(b) R is a 4-methylphenyl group, R²⁴ is a methyl group, R²⁵ is a 2-dimethylaminoethyl group, and the configuration is (2R,3R)-configuration; or
(c) R is a 4-methoxyphenyl group, R²⁴ is a chlorine atom, R²⁵ is a 3-[4-(2-methoxyphenyl)-1-piperazinyl]propyl group, and the configuration is (2S,3S)-configuration.

The pharmaceutically acceptable salt of the optically active cis-3-acetoxy-1,5-benzothiazepine compound (XV) includes, for example, an inorganic acid-addition salt (e.g., hydrochloride, sulfate, phosphate, hydrobromide), and an organic acid-addition salt (e.g., methanesulfonate, acetate, fumarate, maleate, oxalate, benzenesulfonate).

The silylketene acetal compound (II) of the present invention, wherein one of X¹ and X² is a hydrogen atom, and the other is a chlorine atom or a bromine atom, may be prepared, for example, by the method disclosed in Liebigs Annalen der Chemie, p. 687 (1987), i.e., by reacting a haloacetic acid ester compound of the formula (XVI):

X⁵-CH₂-CO₂R⁴ (XVI)

wherein X⁵ is a chlorine atom or a bromine atom, and R⁴ is the same as defined above, with a triflate compound of the formula (XVII): 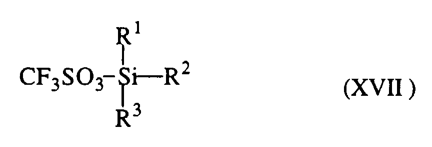 wherein R¹, R² and R³ are the same as defined above, in the presence of a base (e.g., triethylamine, diisopropylethylamine, dimethylethylamine, tetramethylethylenediamine) in a suitable solvent such as ethers (e.g., dioxane, tetrahydrofuran, diethyl ether), nitriles (e.g., acetonitrile), aromatic hydrocarbons (e.g., benzene, toluene), halogenated aliphatic hydrocarbons (e.g., methylene chloride, chloroform, carbon tetrachloride, 1,2-dichloroethane) or without a solvent, at a temperature between 0°C and room temperature.

The silylketene acetal compound (II) wherein both of X¹ and X² are a chlorine atom or a bromine atom may be prepared, for example, by reacting a trihalogenoacetic acid ester compound of the formula (XVIII): 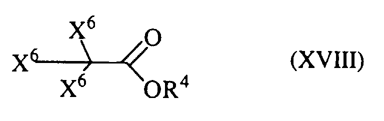 wherein X⁶ is a chlorine atom or a bromine atom, and R⁴ is the same as defined above, with a silane compound of the formula (XIX): 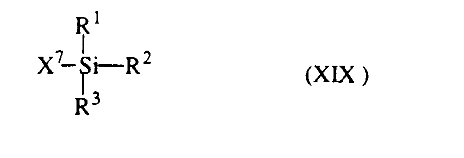 wherein X⁷ is a halogen atom or a trilfluoromethanesulfonyloxy group, and R¹, R² and R³ are the same as defined above, in a suitable solvent (e.g., ethers such as tetrahydrofuran), in the presence of zinc at a temperature between room temperature and a refluxing temperature of the solvent to be used.

The chiral titanium compound (IV) used in the present invention may be prepared, for example, by the method disclosed in Chimia , **45**, 238 (1991), J. Org. Chem., **60**, 1788 (1995), ibid. **57**, 6825 (1992), or Helvetica Chimica Acta, **70**, 954 (1987), i.e., by reacting a Grignard reagent of the formula (XX):

Br-Mg-R⁷ (XX)

wherein R⁷ is the same as defined above, with a chiral dimethoxycarbonyldioxolane compound of the formula (XXI): 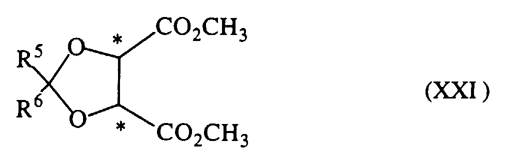 wherein R⁵, R⁶ and * are the same as defined above, in a suitable solvent such as ethers (e.g., tetrahydrofuran, diethyl ether) or aromatic hydrocarbons (e.g., benzene, toluene, xylene), at a temperature between 0°C and 100°C, then reacting the resulting chiral dihydroxymethyldioxolane compound of the formula (XXII): 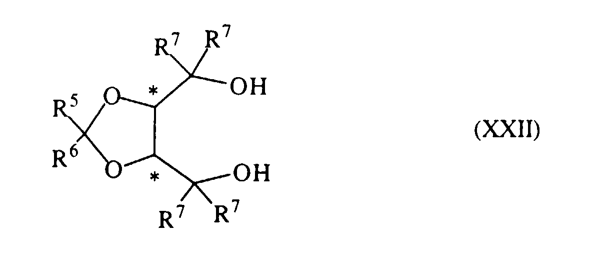 wherein R⁵, R⁶, R⁷ and * are the same as defined above, with a titanium complex of the formula (XXIII):

Ti(R^{8a})ₘ(R^{8b})₄₋ₘ (XXIII)

wherein m is 0, 1, 2, 3 or 4, and R^{8a} and R^{8b} are the same as defined above, and if necessary, together with a silane compound of the formula (XXIV):

Si(R^{8c})₄ (XXIV)

wherein R^{8c} is a halogen atom, at a temperature between 0°C and 50°C in a suitable solvent such as aromatic hydrocarbons (e.g., benzene, toluene, xylene, mesitylene).

The chiral boron compound of the formula (VII) used in the present invention may be prepared, for example, by reacting a carboxylic acid compound of the formula (XXV): 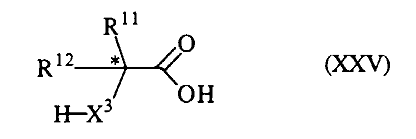 wherein R¹¹ R¹² and X³ are the same as defined above, with a borane (BH₃) in a suitable solvent (e.g., ethers such as tetrahydrofuran, halogenated aliphatic hydrocarbons such as methylene chloride) at a room temperature.

The other chiral Lewis acids used in the present invention may be prepared, for example, by the methods disclosed in Chemistry Letters, 807-810 (1990); Chemistry Express, 5, 335-338 (1991); J. Am. Chem. Soc., **116**, 8837-8838 (1994); J. Am. Chem. Soc., **117**, 2363-2364 (1995); J. Org. Chem., **56**, 2276-2278 (1991); J. Am. Chem. Soc., **113**, 9365-9366 (1991); Syntlett, 439-440 (1991); Tetrahedron Letters, 27, 4721-4724 (1986); J. Am. Chem. Soc., **118**, 5814-5815 (1996); Chemistry and Industry, 824 (1986); and J. Am. Chem. Soc., **113**, 4247-4252 (1991).

Throughout the specification and the claims, the lower alkyl group means a straight chain or branched chain alkyl group having 1 to 6 carbon atoms. The lower alkylene group means a straight chain or branched chain alkylene group having 1 to 6 carbon atoms. The lower alkoxy group means a straight chain or branched chain alkoxy group having 1 to 6 carbon atoms. The lower alkenyl group means a straight chain or branched chain alkenyl group having 2 to 7 carbon atoms. The cycloalkyl group and the cycloalkane ring mean a cycloalkyl group or a cycloalkane ring having 3 to 8 carbon atoms, respectively. The halogen atom is chlorine atom, bromine atom, fluorine atom or iodine atom. The counter anion is, for example, SbF₆⁻, CN⁻, Cl⁻, Br⁻, CH₃COO⁻, ClO₄⁻, CF₃SO₃⁻.

The present invention is illustrated in more detail by the following Examples and Reference Examples, but should not be construed to be limited thereto.

### Example 1

To a solution of (4S,5S)-2,2-dimethyl-α,α,α',α'-tetrakis(p-tolyl)-1,3-dioxolane-4,5-dimethanol (313 mg) in toluene (5 ml) is added dropwise a solution of orthotitanic acid tetraisopropyl ester (170 mg) in toluene (1 ml) at room temperature under argon atmosphere, and the mixture is stirred at the same temperature for 30 minutes. The solution is concentrated under reduced pressure at room temperature, and the resulting colorless solid is dissolved in toluene (5 ml) under argon atmosphere. To the mixture is added dropwise a solution of silicon tetrachloride (306 mg) in toluene (1.5 ml), and the mixture is stirred at room temperature for 30 minutes. The mixture is concentrated under reduced pressure at room temperature to give a chiral titanium compound of the following formula as a red solid. 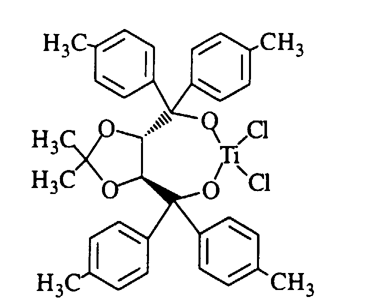 The above chiral titanium compound is dissolved in toluene (5 ml) under argon atmosphere, and the solution thus obtained is used in the subsequent reaction as it is.

The solution of the chiral titanium compound in toluene is cooled to -78°C, and thereto is added dropwise a solution of p-anisaldehyde (68 mg) in toluene (1 ml), and the mixture is stirred at the same temperature for 15 minutes. To the mixture is added dropwise a solution of 2-chloro-1-methoxy-1-trimethylsilyloxyethene (135 mg) in toluene (1 ml), and the mixture is stirred at the same temperature for one hour. To the reaction mixture is added a saturated aqueous sodium chloride solution (30 ml), and the mixture is extracted with ethyl acetate (30 ml). The aqueous layer is extracted again with ethyl acetate (30 ml). The ethyl acetate layers are combined, dried over anhydrous magnesium sulfate, and concentrated under reduced pressure to remove the solvent. The residue thus obtained is dissolved in tetrahydrofuran (2 ml), and thereto is added 1N hydrochloric acid (1 ml), and the mixture is stirred at room temperature for five minutes. To the mixture is added a saturated aqueous sodium chloride solution (30 ml), and the mixture is extracted with ethyl acetate (30 ml). The aqueous layer is extracted again with ethyl acetate (30 ml). The organic layers are combined, dried over anhydrous magnesium sulfate, and concentrated under reduced pressure to remove the solvent. The resulting residue is purified by silica gel column chromatography (solvent; hexane:ethyl acetate = 8:1 → 3:1) to give a mixture (63 mg) of the threo-form [(2R,3S)-isomer and (2S,3R)-isomer] and the erythro-form [(2S,3S)-isomer and (2R,3R)-isomer] (threo:erythro = 1:16) of 2-chloro-3-hydroxy-3-(4-methoxyphenyl)propionic acid methyl ester, as colorless crystals. The ratio of (3S)-compound [(2R,3S)-isomer and (2S,3S)-isomer] and (3R)-compound [(2R,3R)-isomer and (2S,3R)-isomer] in the mixture is 93.0 : 7.0, as determined by HPLC.
M.p. 63-65°C
¹H-NMR (CDCl₃, δ): 2.94 (1H, brs), 3.81 (6H, s), 4.36 (0.94H, d, J=8.0, erythro-form), 4.42 (0.06H, d, J=6.7, threo-form), 5.01 (0.94 H, d, J=8.0, erythroform), 5.07 (0.06H, d, J=6.7, threo-form), 6.86-6.97 (2H, m), 7.25-7.38 (2H, m),
Conditions for HPLC:
Column: CHIRALCEL OJ (4.6 x 250 mm), manufactured by Daicel Chemical Industries, Ltd.
Solvent: n-hexane : ethanol = 90 : 10
Flow rate: 1.0 ml/min.
UV detection: 254 nm
Column temperature: 35°C

### Example 2

To a solution of (4R,5R)-2,2-dimethyl-α,α,α',α'-tetrakis(o-tolyl)-1,3-dioxolane-4,5-dimethanol (313 mg) in toluene (5 ml) is added dropwise a solution of orthotitanic acid tetraisopropyl ester (170 mg) in toluene (1 ml) at room temperature under argon atmosphere, and the mixture is stirred at the same temperature for 30 minutes. The solution is concentrated under reduced pressure at room temperature, and the resulting colorless solid is dissolved in toluene (5 ml) under argon atmosphere. To the mixture is added dropwise a solution of silicon tetrachloride (306 mg) in toluene (1.5 ml), and the mixture is stirred at room temperature for 30 minutes. The mixture is concentrated under reduced pressure at room temperature to give a chiral titanium compound of the following formula as a red solid. 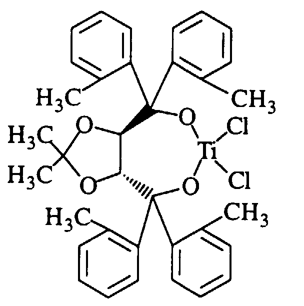 The above chiral titanium compound is dissolved in toluene (5 ml) under argon atmosphere, and the solution thus obtained is used in the subsequent reaction as it is.

The solution of the chiral titanium compound in toluene is cooled to -78°C, and thereto is added dropwise a solution of p-anisaldehyde (68 mg) in toluene (1 ml), and the mixture is stirred at the same temperature for 15 minutes. To the mixture is added dropwise a solution of 2-chloro-1-methoxy-1-trimethylsilyloxyethene (135 mg) in toluene (1 ml), and the mixture is stirred at the same temperature for one hour. To the reaction mixture is added a saturated aqueous sodium chloride solution (30 ml), and the mixture is extracted with ethyl acetate (30 ml). The aqueous layer is extracted again with ethyl acetate (30 ml). The ethyl acetate layers are combined, dried over anhydrous magnesium sulfate, and concentrated under reduced pressure to remove the solvent. The residue thus obtained is dissolved in tetrahydrofuran (2 ml), and thereto is added 1N hydrochloric acid (1 ml), and the mixture is stirred at room temperature for five minutes. To the mixture is added a saturated aqueous sodium chloride solution (30 ml), and the mixture is extracted with ethyl acetate (30 ml). The aqueous layer is extracted again with ethyl acetate (30 ml). The organic layers are combined, dried over anhydrous magnesium sulfate, and concentrated under reduced pressure to remove the solvent. The resulting residue is purified by silica gel column chromatography (solvent; hexane:ethyl acetate = 8:1 → 3:1) to give a mixture (98 mg) of the threo-form [(2R,3S)-isomer and (2S,3R)-isomer] and the erythro-form [(2S,3S)-isomer and (2R,3R)-isomer] (threo:erythro = 11:1) of 2-chloro-3-hydroxy-3-(4-methoxyphenyl)propionic acid methyl ester, as colorless crystals. The ratio of (3S)-compound [(2R,3S)-isomer and (2S,3S)-isomer] and (3R)-compound [(2R,3R)-isomer and (2S,3R)-isomer] in the mixture is 78.7 : 21.3, as determined by HPLC.

### Examples 3-14

p-Anisaldehyde, 2-chloro-1-methoxy-1-trimethylsilyloxyethene,and the chiral titanium compounds as listed in Tables 1 and 2 are treated in the same manner as in Example 1 or 2 to give optically active (3S)- or (3R)-2-chloro-3-hydroxy-3-(4-methoxyphenyl)propionic acid methyl esters at the ratios as listed in Tables 1 and 2.

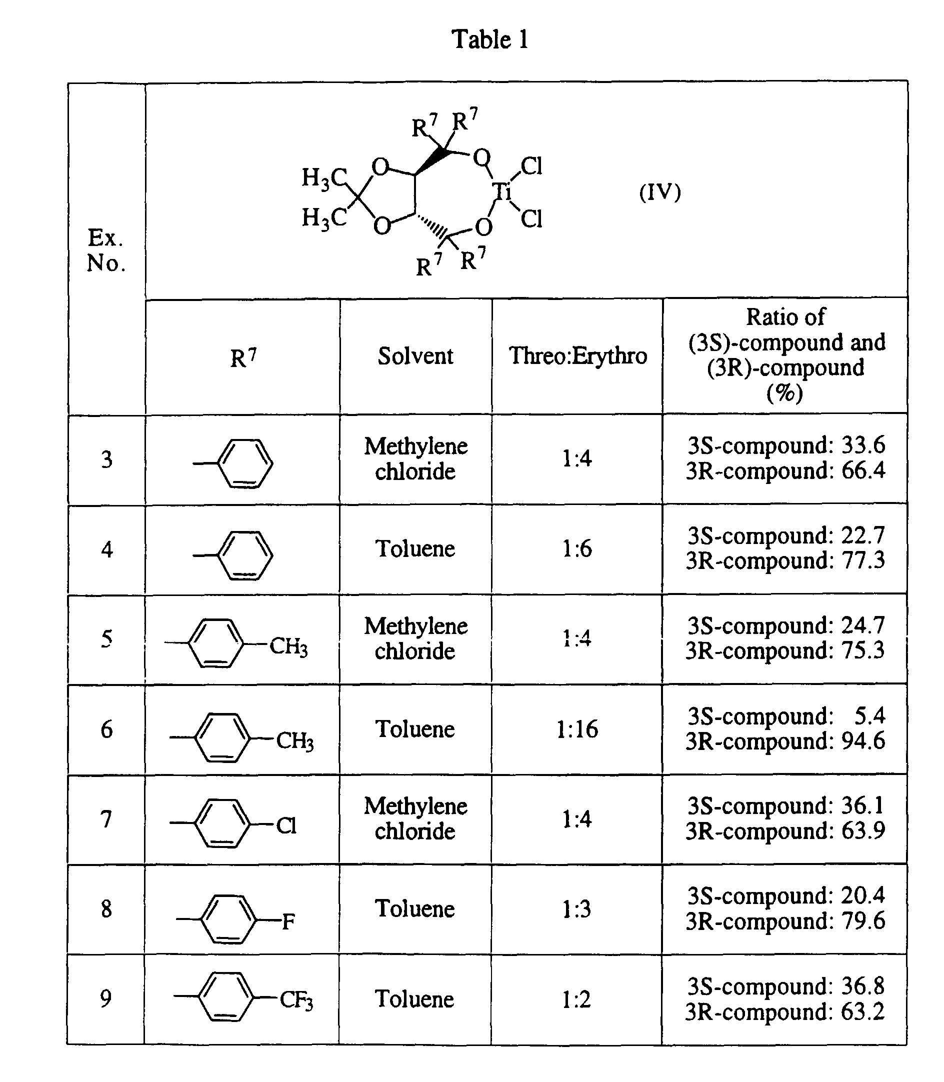

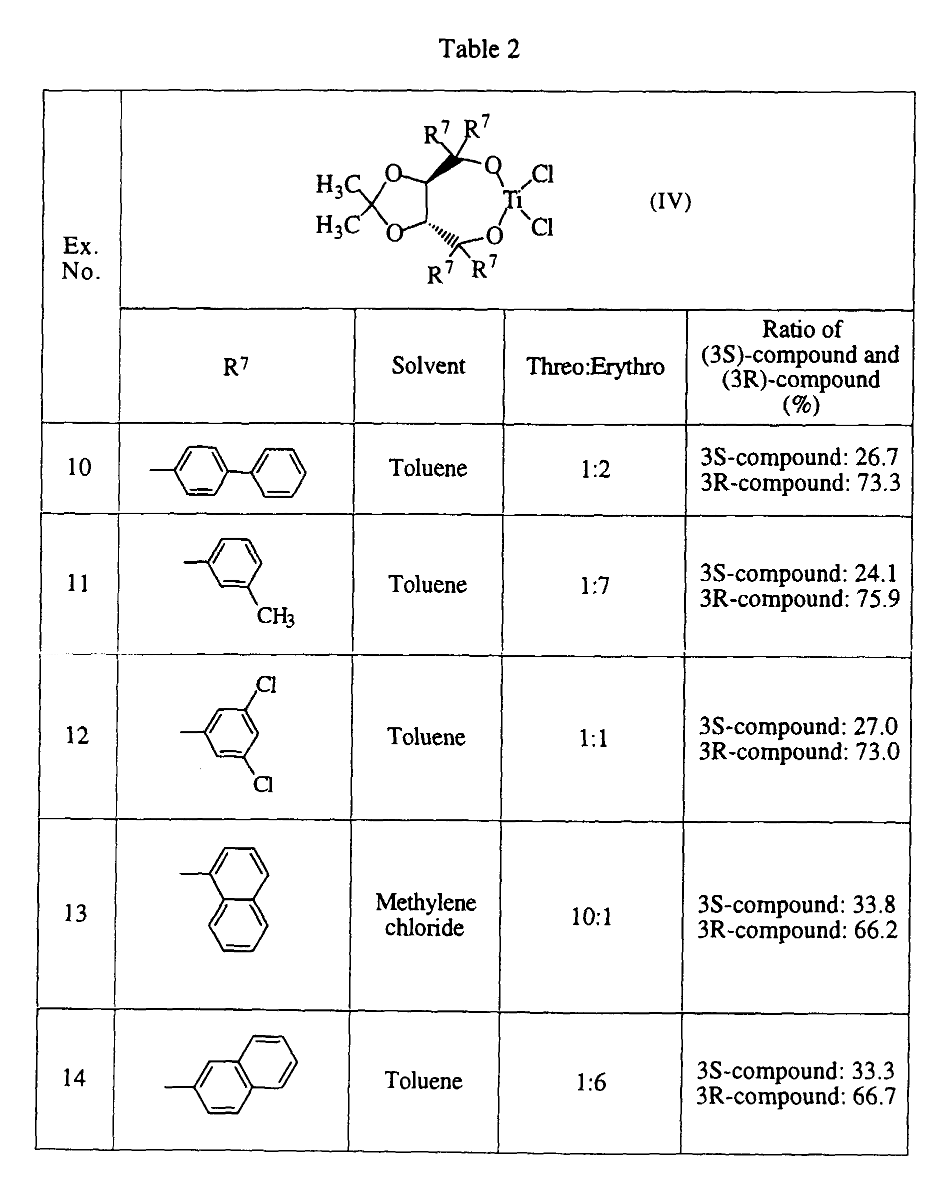

### Example 15

To a solution of N-(p-toluenesulfonyl)-L-tert-leucine (285 mg) in tetrahydrofuran (10 ml) is added dropwise a 1M solution of borane in tetrahydrofuran (1 ml) at room temperature under nitrogen atmosphere, and the mixture is stirred at the same temperature for 30 minutes. The solution is cooled to a temperature below -70°C, and thereto is added dropwise a solution of p-anisaldehyde (136 mg) in tetrahydrofuran (1 ml), and then further thereto is added dropwise a solution of 2-chloro-1-ethoxy-1-trimethylsilyloxyethene (285 mg) in tetrahydrofuran (1 ml). The mixture is stirred at the same temperature for three hours, and thereto is added 0.2N phosphate buffer (pH 7.0, 10 ml), and the mixture is warmed to room temperature. The mixture is separated into an aqueous layer and an organic layer, and the aqueous layer is extracted with ethyl acetate. The organic layers are combined, and washed successively with a saturated aqueous sodium hydrogen carbonate solution and a saturated aqueous sodium chloride solution. The mixture is dried over anhydrous magnesium sulfate, and concentrated under reduced pressure to remove the solvent. The residue thus obtained is dissolved in a mixture of tetrahydrofuran (5 ml) and 2N hydrochloric acid (1 ml), and the mixture is stirred for 30 minutes. The mixture is concentrated under reduced pressure to remove the tetrahydrofuran, and thereto is added ethyl acetate. The mixture is washed successively with a saturated aqueous sodium hydrogen carbonate solution and a saturated aqueous sodium chloride solution, dried over anhydrous magnesium sulfate, and concentrated under reduced pressure. The resulting residue is purified by silica gel column chromatography (solvent; hexane:ethyl acetate = 8:1 → 3:1) to give a mixture (168 mg) of the threo-form [(2R,3S)-isomer and (2S,3R)-isomer] and the erythro-form [(2S,3S)-isomer and (2R,3R)-isomer] (threo:erythro = 67:33) of 2-chloro-3-hydroxy-3-(4-methoxyphenyl)propionic acid ethyl ester, as colorless powder. The ratio of (3S)-compound [(2R,3S)-isomer and (2S,3S)-isomer] and (3R)-compound [(2R,3R)-isomer and (2S,3R)-isomer] in the mixture is 77.3 : 22.7, as determined by HPLC.
¹H-NMR (CDCl₃, δ): 1.15 (2H, t, J=7.1, erythro-form), 1.29 (1H, t, J=7.1, threo-form), 2.91(1H, d, J=3.7), 3.80 (3H, s), 4.10 (1.34H, q, J=7.1 erythro-form), 4.25 (0.67H, q, J=7.1, threo-form), 4.34 (0.33H, d, J=7.9, threo-form), 4.40 (0.67 H, d, J=7.9, erythro-form), 4.99 (0.33H, dd, J=4.3, 7.9, threo-form), 5.07 (0.67H, dd, J=4.3, 7.9, erythro-form), 6.87 (2H, d, J=8.7), 7.30 (2H, d, J=8.7 Hz)
Conditions for HPLC:
Column: CHIRALCEL OJ (4.6 x 250 mm), manufactured by Daicel Chemical Industries, Ltd.
Solvent: n-Hexane: 2-propanol = 80 : 20
Flow rate: 1.0 ml/min.
UV detection: 254 nm
Column temperature: 40°C

### Examples 16-21

p-Anisaldehyde, 2-chloro-1-ethoxy-1-trimethylsilyloxyethene and the chiral boron compounds as listed in Table 3 are treated in the same manner as in Example 15 to give optically active (3S)- and (3R)-2-chloro-3-hydroxy-3-(4-methoxyphenyl)propionic acid ethyl esters at the ratios as listed in Table 3.

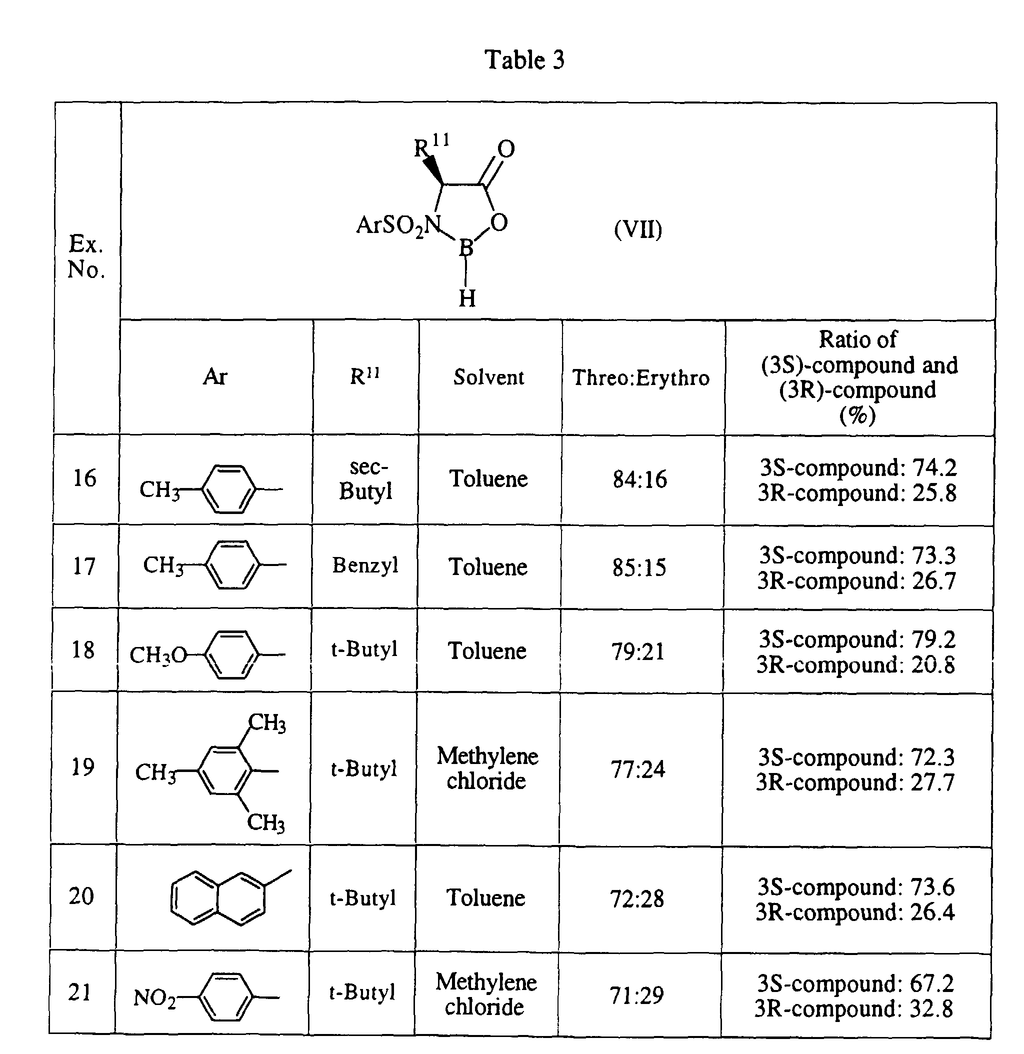

### Example 22

To a solution of N-(p-toluenesulfonyl)-L-valine (271 mg) in methylene chloride (10 ml) is added dropwise a 1M solution of borane in tetrahydrofuran (1 ml) at room temperature under nitrogen atmosphere, and the mixture is stirred at the same temperature for 30 minutes. The solution is cooled to a temperature below -70°C, and thereto is added dropwise a solution of p-anisaldehyde (136 mg) in methylene chloride (1 ml), and then further thereto is added dropwise a solution of 2,2-dichloro-1-methoxy-1-trimethylsilyloxyethene (237 mg) in methylene chloride (1 ml). The mixture is stirred at the same temperature for 9 hours, and thereto is added 0.2N phosphate buffer (pH 7, 10 ml), and the mixture is warmed to room temperature. The mixture is separated into an aqueous layer and an organic layer, and the aqueous layer is extracted with ethyl acetate. The organic layers are combined, and washed successively with a saturated aqueous sodium hydrogen carbonate solution and a saturated aqueous sodium chloride solution. The mixture is dried over anhydrous magnesium sulfate, and concentrated under reduced pressure to remove the solvent. The residue thus obtained is dissolved in a mixture of tetrahydrofuran (5 ml) and 2N hydrochloric acid (1 ml), and the mixture is stirred for 30 minutes. The mixture is concentrated under reduced pressure to remove the tetrahydrofuran, and thereto is added ethyl acetate. The mixture is washed successively with a saturated aqueous sodium hydrogen carbonate solution and a saturated aqueous sodium chloride solution, dried over anhydrous magnesium sulfate, and concentrated under reduced pressure. The resulting residue is purified by silica gel column chromatography (solvent; chloroform:hexane:ethyl acetate = 5:5:1) to give 2,2-dichloro3-hydroxy-3-(4-methoxyphenyl)propionic acid methyl ester (198 mg) as a colorless crystal.

The ratio of (3S)-compound and (3R)-compound is 2.5:97.5.
M.p. 69°C
¹H-NMR (200 MHz, CDCl₃, δ): 3.18 (3H, br), 3.82 (3H, s), 3.91 (3H, s), 5.38 (1H, s), 6.90 (2H, d, J=8.9), 7.45 (2H, d, J=8.7)
Conditions for HPLC:
Column: CHIRALCEL OJ (4.6 x 250 mm), manufactured by Daicel Chemical Industries, Ltd.
Solvent: n-hexane : 2-propanol = 70 : 30
Flow rate: 0.6 ml/min.
UV detection: 230 nm
Column temperature: 35°C

### Example 23

To a solution of N-(o-nitrobenzenesulfonyl)-L-valine (60 mg) in methylene chloride (10 ml) is added dropwise a 1M solution of borane in tetrahydrofuran (0.2 ml) at room temperature under nitrogen atmosphere, and the mixture is stirred at the same temperature for 30 minutes. The solution is cooled to a temperature below -70°C, and thereto are added dropwise a solution of p-anisaldehyde (136 mg) in methylene chloride (1 ml), and a solution of 2,2-dichloro-1-methoxy-1-trimethylsilyloxyethene (237 mg) in methylene chloride (1 ml) simultaneously over a period of 10 hours. The mixture is stirred at the same temperature for 3 hours, and thereto is added 0.2N phosphate buffer (pH 7, 10 ml), and the mixture is warmed to room temperature. The mixture is separated into an aqueous layer and an organic layer, and the aqueous layer is extracted with ethyl acetate. The organic layers are combined, and washed successively with a saturated aqueous sodium hydrogen carbonate solution and a saturated aqueous sodium chloride solution. The mixture is dried over anhydrous magnesium sulfate, and concentrated under reduced pressure to remove the solvent. The residue thus obtained is dissolved in a mixture of tetrahydrofuran (5 ml) and 2N hydrochloric acid (1 ml), and the mixture is stirred for 30 minutes. The mixture is concentrated under reduced pressure to remove the tetrahydrofuran, and thereto is added ethyl acetate. The mixture is washed successively with a saturated aqueous sodium hydrogen carbonate solution and a saturated aqueous sodium chloride solution, dried over anhydrous magnesium sulfate, and concentrated under reduced pressure. The resulting residue is subjected to quantative analysis by HPLC to confirm the production of 2,2-dichloro-3-hydroxy-3-(4-methoxyphenyl)propionic acid methyl ester (206 mg).

The ratio of (3S)-compound and (3R)-compound is 19:81.

### Examples 24-37

p-Anisaldehyde, 2,2-dichloro-1-methoxy-1-trimethylsilyloxyethene and the chiral boron compounds as listed in Tables 4 and 5 are treated in the same manner as in Example 22 or 23 to give optically active (3S)- or (3R)-2,2-dichloro-3-hydroxyl-3-(4-methoxyphenyl)propionic acid methyl esters at the ratios as listed in Tables 4 and 5.

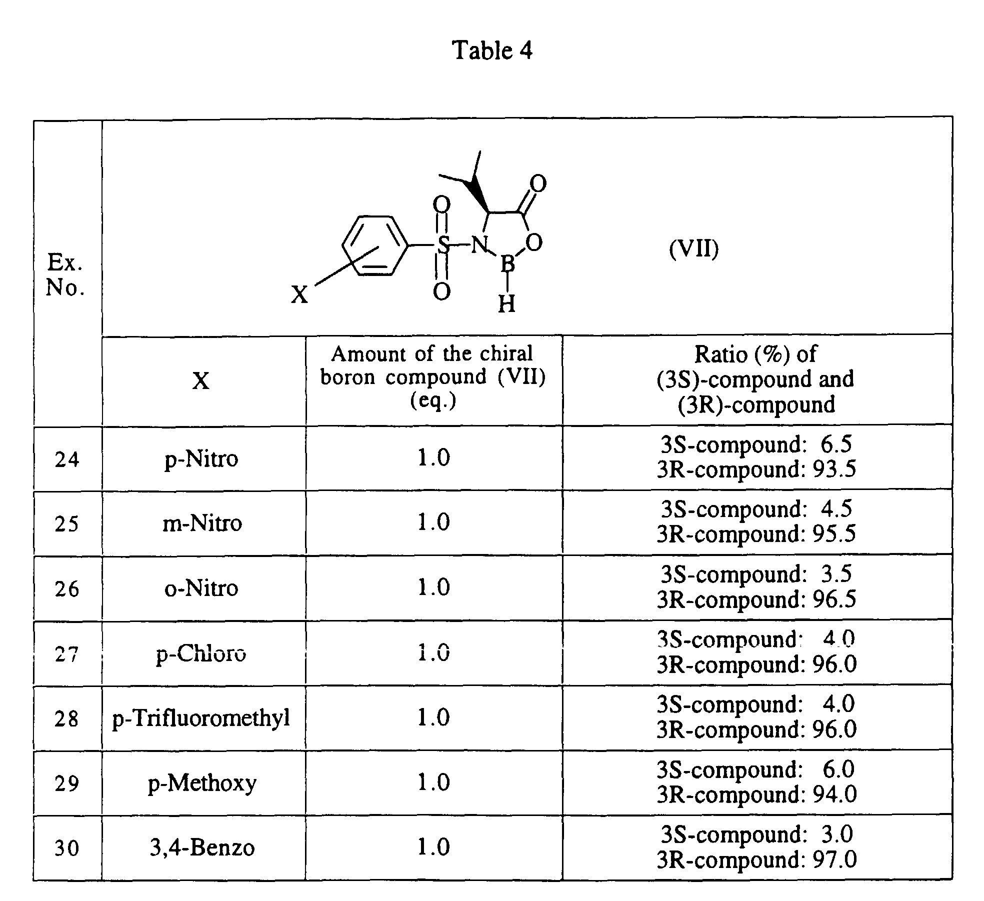

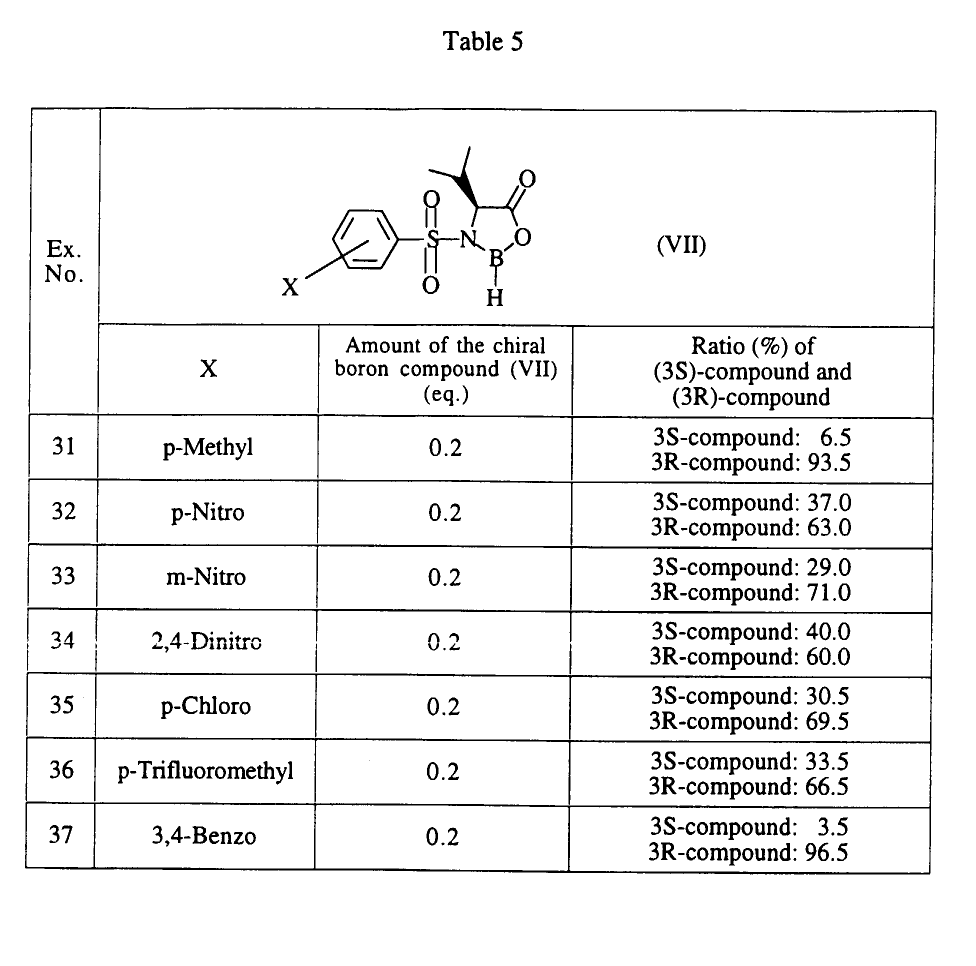

### Reference Example 1

To a solution of chloroacetic acid methyl ester (5.43 g) in triethylamine (50 ml) is added dropwise trifluoromethanesulfonic acid trimethylsilyl ester (11.1 g) over a period of 30 minutes with ice-cooling under nitrogen atmosphere. The mixture is stirred at the same temperature for two hours, and thereto is added n-pentane (50 ml), and the mixture is allowed to stand at room temperature to separate. About 60 ml of the upper layer is collected, and concentrated under reduced pressure, and the residue is evaporated to give 2-chloro-1-methoxy-1-trimethylsilyloxyethane (2.56 g).
B.p. 62-63°C (5 mmHg)
¹H-NMR (CDCl₃, δ): 0.27 (9H, s), 3.55 (3H, s), 4.55 (1H, s)

### Reference Example 2

To a suspension of magnesium (1.07 g) and a crystal of iodine in anhydrous tetrahydrofuran (50 ml) is added dropwise about 1/5 amount of 3-bromotoluene (7.53 g). The remaining 3-bromotoluene is dissolved in anhydrous tetrahydrofuran (50 ml), and added dropwise into the above mixture. After addition, the mixture is refluxed for 30 minutes to give a Grignard reagent. The Grignard reagent (4.4 equivalents) thus obtained is cooled to 10°C, and thereto is added dropwise a solution of (2R,3R)-2,3-O-isopropylidene-L-tartaric acid dimethyl ester (2.18 g) in anhydrous tetrahydrofuran (10 ml). After addition, the mixture is stirred at the same temperature for two hours, and then stirred at room temperature overnight. The reaction mixture is poured into chilled saturated aqueous ammonium chloride solution (500 ml), and the mixture is extracted with ethyl acetate. The aqueous layer is extracted again with ethyl acetate. The organic layers are combined, and washed with a saturated aqueous sodium chloride solution, dried over anhydrous magnesium sulfate, and concentrated under reduced pressure. The residue is purified by silica gel column chromatography (solvent; hexane:ethyl acetate = 10:1) to give (4R,5R)-2,2-dimethyl-α,α,α',α'-tetrakis(m-tolyl)-1,3-dioxolane-4,5-dimethanol (1.3 g) as a colorless powder.
IR (nujol): 3300, 2930, 1460, 1380 (cm⁻¹)
¹H-NMR (CDCl₃, δ): 1.06 (6H, s), 2.26 (6H, s), 2.33 (6H, s), 3.84 (2H, s), 4.56 (2H, s), 7.00-7.40 (16H, m)
MS (m/z): 522.5 (M⁺), 521.6 (M-H)
[α]_{D}²⁴: -53.7° (c=1.00, chloroform)

### Reference Example 3

To a suspension of magnesium (535 mg) and a crystal of iodine in anhydrous tetrahydrofuran (25 ml) is added dropwise about 1/5 amount of 1-bromo-3,5-dichlorobenzene (4.85 g). The remaining 1-bromo-3,5-dichlorobenzene is dissolved in anhydrous tetrahydrofuran (25 ml), and added dropwise into the above mixture. After addition, the mixture is refluxed for 30 minutes to give a Grignard reagent. The Grignard reagent (4.4 equivalents) thus obtained is cooled to 10°C, and thereto is added dropwise a solution of (2R,3R)-2,3-O-isopropylidene-L-tartaric acid dimethyl ester (1.09 g) in anhydrous tetrahydrofuran (10 ml). After addition, the mixture is stirred at the same temperature for 50 minutes, and then stirred at room temperature overnight. The reaction mixture is poured into chilled saturated aqueous ammonium chloride solution (500 ml), and the mixture is extracted with ethyl acetate. The aqueous layer is extracted again with ethyl acetate. The organic layers are combined, washed with a saturated aqueous sodium chloride solution, dried over anhydrous magnesium sulfate, and concentrated under reduced pressure. The residue is purified by silica gel column chromatography (solvent; hexane:ethyl acetate = 50:1) to give a colorless powder (1.7 g), which is crystallized from hexane, and then further recrystallized from hexane to give (4R,5R)-2,2-dimethyl-α,α,α',α'-tetrakis(3,5-dichlorophenyl)-1,3-dioxolane-4,5-dimethanol (630 mg) as colorless crystals.
IR (nujol): 3160, 2930, 1580, 1570, 1460 (cm⁻¹)
¹H-NMR (CDCl₃, δ): 1.25 (6H, s), 3.98 (2H, s), 4.38 (2H, s), 7.14 (4H, d, J=1.83), 7.26 (2H, t, J=1.83), 7.35 (2H, t, J=1.83), 7.39 (4H, d, J=1.83)
MS (m/z): 742 (M⁺), 741 (M-H)
[α]_{D}²⁴: -84.3° (c=1.00, chloroform)
M.p. 124.3-126.7°C

### Reference Example 4

To a suspension of zinc (4.9 g) in tetrahydrofuran (50 ml) is added dropwise a mixture of trimethylsilyl chloride (7.6 ml) and dichloroacetic acid methyl ester (7.15 g) at room temperature under nitrogen atmosphere. The mixture is refluxed for one hour. The reaction mixture is cooled to room temperature, and thereto is added hexane (50 ml). The insoluble materials are removed by filtration under reduced pressure, and the filtrate is concentrated. The residue is evaporated under reduced pressure to give 2-chloro-1-methoxy-1-trimethylsilyloxyethene (5.62 g).
B.p. 47-51°C (2 mmHg)
¹H-NMR (CDCl₃, δ): 0.27 (9H, s), 3.55 (3H, s), 4.55(1H,s)

### Reference Example 5

To a suspension of zinc (17.7 g) in tetrahydrofuran (100 ml) is added dropwise a mixture of trimethylsilyl chloride (15.2 ml) and trichloroacetic acid methyl ester (17.7 g) at room temperature under nitrogen atmosphere over a period of 15 minutes, during which the reflux starts along with the proceeding of the reaction. After addition, the reaction mixture is cooled to room temperature, and thereto is added hexane (100 ml). The insoluble materials are removed by filtration under reduced pressure, and the filtrate is concentrated, and the residue is evaporated under reduced pressure to give 2,2-dichloro-1-methoxy-1-trimethylsilyloxyethene (18.3 g).
B.p. 64-65°C (2 mmHg)
¹H-NMR (CDCl₃, δ): 0.31(9H, s), 3.65 (3H, s)

As to the effects of the present invention, (3R)- or (3S)-2-halogeno-3-hydroxy-propionic acid ester compound (III) can be stereoselectively prepared in a single step by reacting the aldehyde compound (I) and the silylketene acetal compound (II) in the presence of a chiral Lewis acid. The process of the present invention does not need either the use of expensive metals and chiral reagents, or complicated procedures, and the process of the present invention does not need to dispose of environmentally hazardous waste water, which is industrially a serious problem. Therefore, the process of the present invention is industrially advantageous for preparing an intermediate for medicaments.

The tenn "lower" used throughout the specification and claims in relation to alkyl and the other specified groups is defined as groups having one to six carbon atoms

## Claims

1. A process for preparing an optically active 2-halogeno-3-hydroxypropionic acid ester of the formula (III): 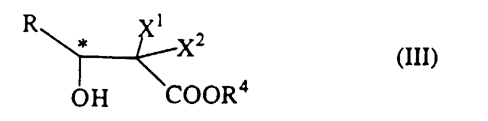 wherein R is a lower alkyl group, a lower alkenyl group, a substituted or unsubstituted aryl group or a cycloalkyl group, R⁴ is a lower alkyl group or a substituted or unsubstituted aryl group, one of X¹ and X² is a hydrogen atom, and the other is a chlorine atom or a bromine atom, or both are a chlorine atom or a bromine atom, and * means an asymmetric carbon atom, which comprises reacting an aldehyde compound of the formula (I):
R-CHO (I)
wherein R is the same as defined above, with a silylketene acetal compound of the formula (II): 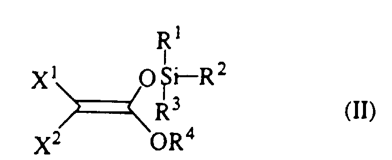 wherein R¹, R² and R³ are the same or different and each are a hydrogen atom, a ]ower alkyl group or a substituted or unsubstituted aryl group, and R⁴, X¹ and X² are the same as defined above, in the presence of a chiral Lewis acid.

2. The process according to claim 1, wherein the chiral Lewis acid is a chiral complex consisting of a ligand, and a boron or a metal selected from titanium, copper, aluminum and tin.

3. The process according to claim 2, wherein the ligand is a member selected from the following compounds:
(1) 1,2,3,4-tetrahydroxybutane having substituents on 1- and 4-positions, wherein 2- and 3-hydroxy groups may be protected;
(2) [1,1'-binaphthalene]-2,2'-diol having optionally substituents;
(3) α-amino acid having a substituent on the carbon atom at α-position, wherein the α-amino group may be protected;
(4) α-hydroxycarboxylic acid having a substituent on the carbon atom at α-position;
(5) 1-(protected or unprotected amino)-2-hydroxycycloalkane having optionally a substituent on 1- and 2-positions;
(6) bridged 1,2-dihydroxycycloalkane having optionally a substituent; and
(7) 1,2-dihydroxycycloalkane having a substituent on 1- and/or 2-positions.

4. The process according to any one of the preceding claims, wherein the chiral Lewis acid is a member selected from the following chiral complexes:
(1) a chiral titanium compound of the formula (IV): 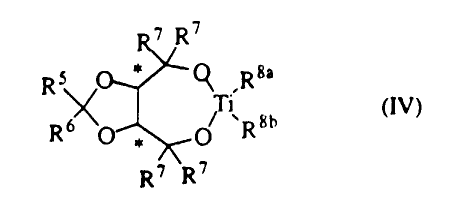 wherein R⁵ and R⁶ are the same or different and each are a hydrogen atom, a lower alkyl group or a substituted or unsubstituted aryl group, or both combine each other to form a lower alkylene group or an oxo group, R⁷ is a lower alkyl group, a substituted or unsubstituted aryl group or a cycloalkyl group, R^{8a} and R^{8b} are the same or different and each are a halogen atom, a trifluoromethane-sulfonyloxy group or a lower alkoxy group, and * means an asymmetric carbon atom;
(2) a chiral titanium compound of the formula (V-a) or (V-b): 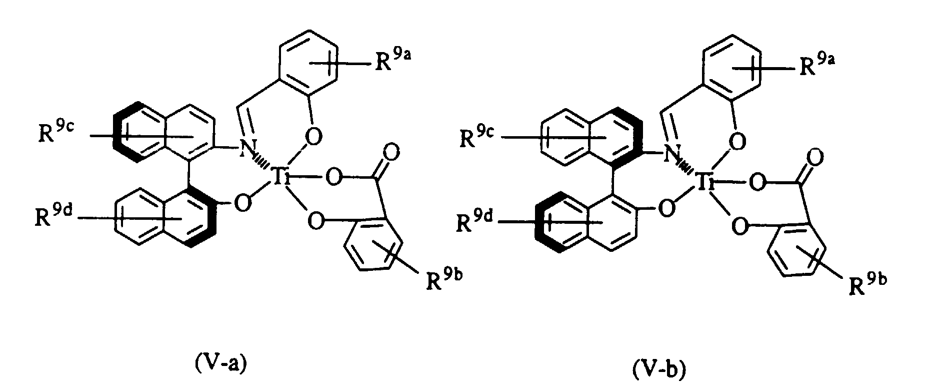 wherein R^{9a}, R^{9b}, R^{9c} and R^{9d} are the same or different and each are a hydrogen atom, 1 or 2 halogen atoms or a lower alkyl group;
(3) a chiral titanium compound of the formula (VI-a) or (VI-b): 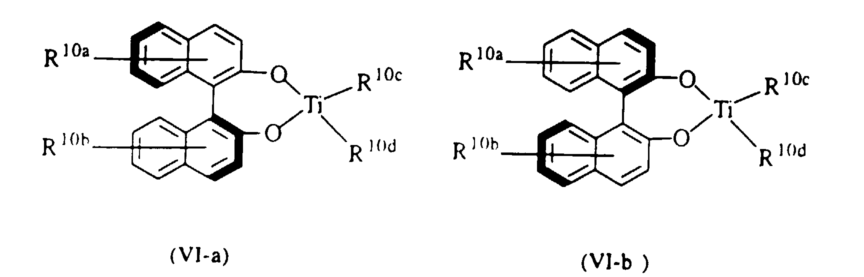 wherein R^{10a} and R^{10b} are the same or different and each are a hydrogen atom, I or 2 halogen atoms or a lower alkyl group, and R^{10c} and R^{10d} are the same or different and each are a halogen atom, a trifluoromethanesulfonyloxy group or a lower alkoxy group;
(4) a chiral boron compound of the formula (VII): 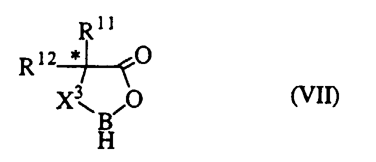 wherein X³ is an oxygen atom, a lower alkylsulfonyl-substituted nitrogen atom, a trifluoromethanesulfonyl-substituted nitrogen atom, or a substituted or unsubstituted arylsulfonyl-substituted nitrogen atom, R¹¹ and R¹² are different, and independently a hydrogen atom, a substituted or unsubstituted lower alkyl group, or a substituted or unsubstituted aralkyl group, or both combine each other to form a substituted or unsubstituted lower alkylene group, and * means an asymmetric carbon atom;
(5) a chiral copper compound of the formula (VIII) : 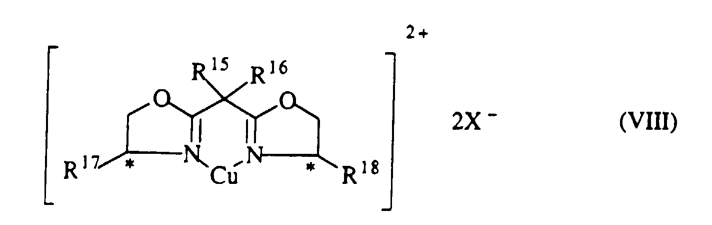 wherein R¹⁵, R¹⁶, R¹⁷ and R¹⁸ are the same or different, and each are a lower alkyl group, X⁻ is a counter anion, and * means an asymmetric carbon atom;
(6) a chiral aluminum compound of the formula (IX): 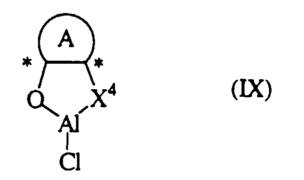 wherein X⁴ is an oxygen atom, or a substituted or unsubstituted arylsulfonyl-substituted nitrogen atom, Ring A is a substituted or unsubstituted bridged cycloalkane ring, a substituted or unsubstituted cycloalkane ring, and * means an asymmetric carbon atom, provided that when X⁴ is an oxygen atom, then Ring A is not an unsubstituted cycloalkane ring; and
(7) a chiral tin compound of the formula (X): 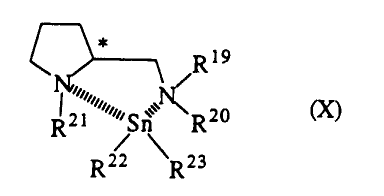 wherein R¹⁹ and R²⁰ are different and independently a hydrogen atom or a substituted or unsubstituted aryl group, or both combine each other to form a substituted or unsubstituted lower alkylene group, R²¹ is a lower alkyl group, R²² and R²³ are a trifluoromethanesulfonyloxy group or a chlorine atom, and * means an asymmetric carbon atom.

5. The process according to claim 4, wherein R⁵ and R⁶ are the same lower alkyl group, R⁷ is a phenyl group or naphthyl group which may optionally be substituted by 1 or 2 groups selected from a halogen atom, a trifluoromethyl group, a phenyl group and a lower alkyl group, and both of R^{8a} and R^{8b} are a halogen atom.

6. The process according to claim 4, wherein X³ is a nitrogen atom substituted by a phenylsulfonyl group or naphthylsulfonyl group which may optionally be substituted by 1 to 3 groups selected from a nitro group, a halogen atom, a lower alkyl group, a halogeno-lower alkyl group and a lower alkoxy group, and R¹¹ and R¹² are different, and independently a hydrogen atom, a lower alkyl group or a benzyl group.

7. The process according to claim 6, wherein R is a 4-methoxyphenyl group, all of R¹, R² and R³ are a methyl group, R⁴ is a methyl group, both of R⁵ and R⁶ are a methyl group, R⁷ is a methylphenyl group, and both of R^{8a} and R^{8b} are a chlorine atom.

8. A process for preparing an optically active trans-2,3-epoxypropionic acid ester compound of the formula (XI): 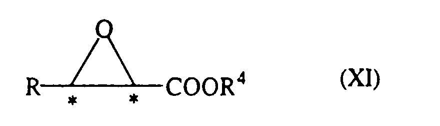 wherein R is a lower alkyl group, a lower alkenyl group, a substituted or unsubstituted aryl group or a cycloalkyl group, R⁴ is a lower alkyl group or a substituted or unsubstituted aryl group, and * means an asymmetric carbon atom,
which comprises preparing the optically active 2-halogeno-3-hydroxypropionic acid ester compound of the formula (III): 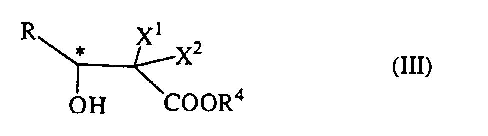 wherein one of X¹ and X² is a hydrogen atom, and the other is a chlorine atom or a bromine atom, or both are a chlorine atom or a bromine atom, and R, R⁴ and *are the same as defined above by a process according to any one of claims 1 to 7, and then subjecting the compound of formula (III) to intramolecular cyclization reaction, provided that when both of X' and X² are a chlorine atom or a bromine atom, the compound (III) is subjected to reduction reaction in order to convert one of X¹ and X² into a hydrogen atom, prior to the intramolecular cyclization reaction.

9. A process for preparing an optically active cis-3-acetoxy-1,5-benzothiazepine compound of the formula (XV): 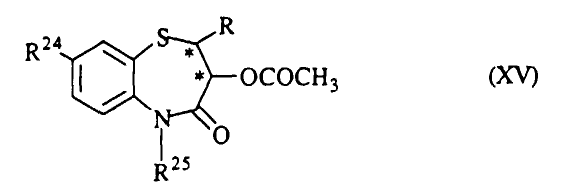 wherein R is a lower alkyl group, a lower alkenyl group, a substituted or unsubstituted aryl group or a cycloalkyl group, R²⁴ is a hydrogen atom, a halogen atom or a lower alkyl group, R²⁵ is a 2-dimethylaminoethyl group or a 3-[4-(2-methoxyphenyl)-1-piperazinyl]propyl group, and * means an asymmetric carbon atom, or a pharmaceutically acceptable salt thereof, which comprises
(i) preparing the optically active trans-2,3-epoxypropionic acid ester compound of the formula (XI): 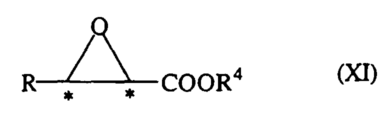 wherein R⁴ is a lower alkyl group or a substituted or unsubstituted aryl group, R and * are the same as defined above, by the process according to claim 8, and then reacting the compound of formula (XI) with an aminothiophenol compound of the formula (XII-a): 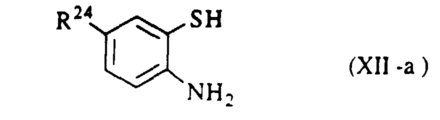 wherein R²⁴ is a hydrogen atom, a halogen atom or a lower alkyl group, or with a nitrothiophenol compound of the formula (XII-b): 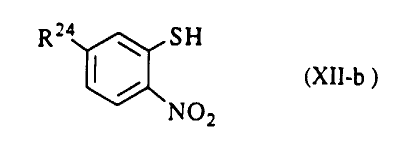 wherein R²⁴ is the same as defined above, and reducing the nitro group of the product,
(ii) if necessary, hydrolyzing the resulting optically active threo-3-(2-aminophenylthio)-2-hydroxypropionic acid ester compound of the formula (XIII-a): 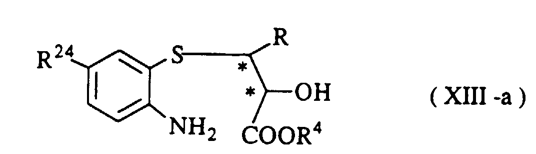 wherein R, R⁴, R²⁴ and * are the same as defined above,
(iii) subjecting the product obtained by the hydrolysis of the compound (XIII-a), or the compound (XIII-a) per se, to intramolecular cyclization reaction to give an optically active cis-3-hydroxy-1,5-benzothiazepine compound of the formula (XIV-a): 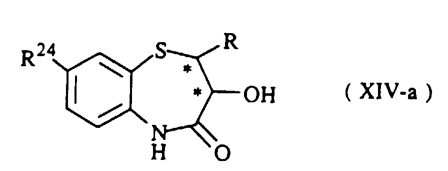 wherein R, R²⁴ and * are the same as defined above,
(iv) introducing a 2-dimethylaminoethyl group or a 3-[4-(2-methoxyphenyl)-1-piperazinyl]propyl group onto the 5-nitrogen atom, and acetylating the 3-hydroxy group of the compound (XIV-a), or vice verse,
(v) if necessary, followed by converting the product into a pharmaceutically acceptable salt thereof.

10. A process for preparing an optically active cis-3-acetoxy-1,5-benzothiazepine compound of the formula (XV): 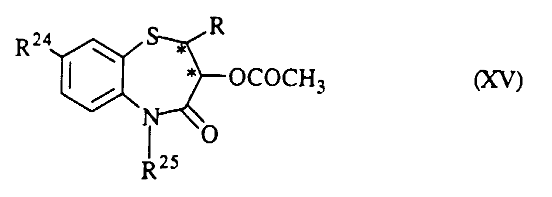 wherein R is a lower alkyl group, a lower alkenyl group, a substituted or unsubstituted aryl group or a cycloalkyl group, R²⁴ is a hydrogen atom, a halogen atom or a lower alkyl group, R²⁵ is a 2-dimethylaminoethyl group or a 3-[4-(2-methoxyphenyl)-1-piperazinyl]propyl group, and * means an asymmetric carbon atom, or a pharmaceutically acceptable salt thereof, which comprises
(i) preparing the optically active trans-2,3-epoxypropionic acid ester compound of the formula (XI): 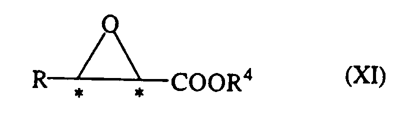 wherein R⁴ is a lower alkyl group or a substituted or unsubstituted aryl group, and R and * are the same as defined above, by the process according to claim 8, and then reacting the compound of formula (XI) with an N-alkylaminothiophenol compound of the formula (XII-c): 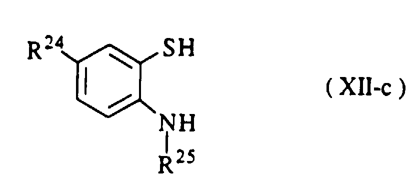 wherein R²⁴ and R²⁵ are the same as defined above,
(ii) if necessary, hydrolyzing the resulting optically active threo-3-[2-(N-alkylamino)phenylthio]-2-hydroxypropionic acid ester compound of the formula (XIII-b): 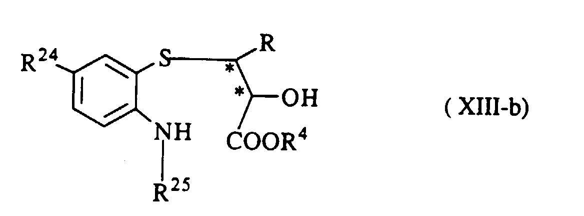 wherein R, R⁴, R²⁴, R²⁵ and * are the same as defined above,
(iii) subjecting the product obtained by the hydrolysis of the compound (XIII-b), or the compound (XIII-b) per se, to intramolecular cyclization reaction to give an optically active cis-5-alkyl-3-hydroxy-1,5-benzothiazepine compound of the formula (XIV-b): 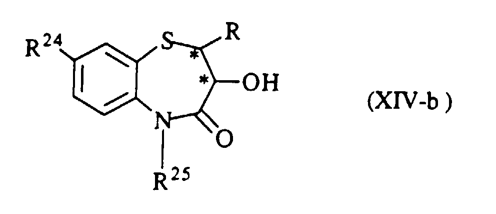 wherein R, R²⁴, R²⁵ and * are the same as defined above,
(iv) acetylating the 3-hydroxy group of the compound (XIV-b),
(v) if necessary, followed by converting the product into a pharmaceutically acceptable salt thereof.

11. The process according to any one of the preceding claims, wherein R is a lower alkylphenyl group or a lower alkoxyphenyl group, R¹, R² and R³ are the same lower alkyl group, and R⁴ is a lower alkyl group.

12. The process according to any one of the preceding claims, wherein the configuration of the 3-position of the optically active 2-halogeno-3-hydroxy-propionic acid ester compound (III) is R-configuration.

13. The process according to any one of claims 1 to 11, wherein the configuration of the 3-position of the optically active 2-halogeno-3-hydroxypropionic acid ester compound (III) is S-configuration.
